(19) **Europäisches Patentamt**

**European Patent Office**

**Office européen des brevets**

(11) **EP 0 774 297 B1**

(12) **EUROPÄISCHE PATENTSCHRIFT**

(45) Veröffentlichungstag und Bekanntmachung des Hinweises auf die Patenterteilung:
**09.02.2000 Patentblatt 2000/06**

(51) Int Cl.⁷: **B01J 23/88**

(21) Anmeldenummer: **96117834.0**

(22) Anmeldetag: **07.11.1996**

(54) **Multimetalloxide**

Multi-metal oxides

Oxydes multimétalliques

(84) Benannte Vertragsstaaten:
**BE DE DK ES FR GB IT NL**

(30) Priorität: **16.11.1995 DE 19542755**

(43) Veröffentlichungstag der Anmeldung:
**21.05.1997 Patentblatt 1997/21**

(73) Patentinhaber: **BASF AKTIENGESELLSCHAFT**
**67056 Ludwigshafen (DE)**

(72) Erfinder:
• **Hibst, Hartmut, Prof.Dr.**
**69198 Schriesheim (DE)**
• **Tenten, Andreas, Dr.**
**67487 Maikammer (DE)**
• **Marosi, Laszlo, Dr.**
**67063 Ludwigshafen (DE)**

(56) Entgegenhaltungen:
**EP-A- 0 293 859**     **EP-A- 0 575 897**
**EP-A- 0 609 750**     **EP-A- 0 668 102**
**US-A- 4 208 306**

## Beschreibung

Vorliegende Erfindung betrifft Multimetalloxide der allgemeinen Formel I

$$Mo_{12-a-b-c} V_a M^1_b M^2_c O_x \tag{I},$$

in der die Variablen folgende Bedeutung haben:

$M^1$ = W und/oder Nb,
$M^2$ = Ti, Zr, Hf, Ta, Cr, Si und/oder Ge,
a = 0,1 bis 6, bevorzugt 0,5 bis 4,5,
b = 0 bis 6, vorzugsweise 0,1 bis 6 und besonders bevorzugt 0,5 bis 4,
c = 0 bis 6, häufig 0,1 bis 6 oder 0,5 bis 4,

mit der Maßgabe, daß a + b + c = 0,1 bis 6, bevorzugt 0,5 bis 4,5 und

x = eine Zahl, die durch die Wertigkeit und Häufigkeit der von Sauerstoff verschiedenen Elemente in I bestimmt wird,

die dadurch gekennzeichnet sind, daß
ihre atomare Raumanordnung unter Anwendung von Cu-K$\alpha$-Strahlung ($\lambda$ = 1,54178 Å) ein Pulver-Röntgenbeugungsspektrum (die Intensität A der gebeugten Röntgenstrahlung aufgetragen als Funktion des zweifachen Beugungswinkels $(2\ominus)$) bedingt, das im $2\ominus$-Bereich (angegeben in der Einheit für ebene Winkel: °=Grad) von 5° bis 50° mindestens die nachfolgenden charakteristischen Beugungslinien $A^1$, $A^3$, $A^5$, $A^9$ und $A^{10}$, jedoch höchstens die nachfolgenden Beugungslinien $A^1$ bis $A^{10}$ enthält:

| Röntgenbeugungslinie | $2\ominus$ [°] |
|---|---|
| $A^1$ | $8,3 \pm 0,7$ |
| $A^2$ | $14,4 \pm 0,7$ |
| $A^3$ | $22,3 \pm 0,2$ |
| $A^4$ | $23,5 \pm 0,7$ |
| $A^5$ | $27,2 \pm 0,4$ |
| $A^6$ | $32,0 \pm 0,8$ |
| $A^7$ | $34,8 \pm 0,6$ |
| $A^8$ | $38,7 \pm 0,5$ |
| $A^9$ | $45,4 \pm 0,4$ |
| $A^{10}$ | $48,8 \pm 0,4$ |

Dabei sind die Wellenlänge $\lambda$ der zur Beugung verwendeten Röntgenstrahlung und der Beugungswinkel $\ominus$ über die Bragg'sche Beziehung miteinander verknüpft:

$$2 \sin \ominus = \lambda/d,$$

wobei d der zur jeweiligen Beugungslinie gehörige Netzebenenabstand der atomaren Raumanordnung ist.
Ferner betrifft vorliegende Erfindung Verfahren zur Herstellung von Multimetalloxiden I sowie ihre unmittelbare Verwendung als Aktivmasse von Katalysatoren für die katalytische Gasphasenoxidation organischer Verbindungen.
Auch betrifft vorliegende Erfindung die Verwendung von Multimetalloxiden I als Ausgangsverbindungen zur Herstellung von Mo und V enthaltenden Multimetalloxidmassen, die ihrerseits als Aktivmasse für die katalytische Gasphasenoxidation organischer Verbindungen geeignet sind.
Die Herstellung von Mo und V enthaltenden Multimetalloxidmassen sowie deren Verwendung als Aktivmassen von Katalysatoren für die katalytische Gasphasenoxidation organischer Verbindungen (z.B. Acrolein zu Acrylsäure) ist allgemein bekannt (z.B. EP-A 293 859).

Anfänglich erfolgte die Herstellung solcher Mo und V enthaltenden Multimetalloxidmassen dadurch, daß man von jedem ihrer elementaren Konstituenten eine geeignete Quelle (Ausgangsverbindung) bereitstellte, aus der Gesamtmenge dieser Quellen ein möglichst inniges, vorzugsweise feinteiliges, der geforderten Stöchiometrie der gewünschten Multimetalloxidmasse entsprechend zusammengesetztes, Trockengemisch erzeugte, und dieses bei erhöhter Temperatur mehrere Stunden unter Inertgas oder unter Sauerstoff enthaltendem Gas (z.B. Luft) calcinierte ("Eintopfverfahren"). Wesentlich für die Quellen der elementaren Konstituenten der Multimetalloxidmassen war dabei lediglich, daß es sich entweder bereits um Oxide handelte oder um solche Verbindungen, die durch Erhitzen, wenigstens in Anwesenheit von Sauerstoff, in Oxide überführbar sind (vgl. z.B. DE-A 4 335 973 und die US-A 4 035 262).

Inzwischen ist bekannt (vgl. z.B. EP-A 835, DE-C 3 338 380, DE-A 4 220 859, DE-A 4 307 381, DE-A 4 405 085, DE-A 4 405 059, DE-A 4 405 060, DE-A 4 405 514 und DE-A 44 40 891) daß es günstig sein kann, zunächst ein lediglich eine Untermenge der Gesamtmenge der elementaren Konstituenten der gewünschten Multimetalloxidmasse umfassendes Multimetalloxid getrennt vorzubilden und dieses getrennt vorgebildete Untermengenmultimetalloxid anschließend als feinteilige Quelle zur Erzeugung der gewünschten Multimetalloxidmasse einzusetzen.

In der Regel resultieren dabei Multimetalloxidmassen mit mehrphasigem Aufbau, die für die katalytische Gasphasenoxidation organischer Verbindungen teilweise vorteilhafter sind, als die nach dem an sich bekannten Eintopfverfahren hergestellten Multimetalloxidmassen.

Aufgabe der vorliegenden Erfindung war es, neue Untermengenmetalloxide von Mo und V enthaltenden Multimetalloxidmassen zur Verfügung zu stellen, die als Ausgangsverbindungen zur Herstellung von mehrphasigen Mo- und V-haltigen Multimetalloxidmassen, die sich insbesondere als Aktivmasse für die gasphasenkatalytische Oxidation von Acrolein zu Acrylsäure eignen, verwendbar sind.

Demgemäß wurden die eingangs definierten Multimetalloxide I gefunden. Günstige Multimetalloxide I sind u. a. jene mit x = 15 bis 50. Vorzugsweise beträgt x 25 bis 40 und besonders bevorzugt 30 bis 40. Ferner eignen sich von den Multimetalloxiden I u.a. diejenigen mit c = 0. Im Fall c = 0 und $M^1$ = ausschließlich W sind Multimetalloxide I geeignet, deren Vanadium zu $\geq 25$, oder $\geq 50$, oder $\geq 75$, oder $\geq 95$, oder $\geq 99$ % als $V^{4+}$ vorliegt.

Typisch für die neuen Multimetalloxide I ist, daß ihr Pulver-Röntgenbeugungsspektrum unter Anwendung der Cu-Kα-Strahlung im 20-Bereich von 5° bis 50° als kennzeichnenden Fingerabdruck mindestens die Beugungslinien $A^1$, $A^3$, $A^5$, $A^9$ und $A^{10}$, jedoch keinesfalls mehr als die Beugungslinien $A^1$, $A^2$, $A^3$, $A^4$, $A^5$, $A^6$, $A^7$, $A^8$, $A^9$ und $A^{10}$ enthält.

Bemerkenswerterweise handelt es sich bei einem Teil der Beugungslinien um solche, die eine relativ große Halbwertsbreite FWHM (die Breite der Beugungslinie (die Intensität aufgetragen als Funktion von 2⊖) auf der halben Höhe ihrer maximalen Amplitude, angegeben in der Einheit für ebene Winkel) aufweisen, was eine außergewöhnliche Ausprägung von Nah- und Fernordnung in den neuen Multimetalloxiden I widerspiegelt (vgl. Beispiele). Diese besondere Gestaltung von Nah- und Fernordnung zeichnet nach diesseitiger Auffassung u.a. für die besonderen katalytischen Eigenschaften der erfindungsgemäßen Multimetalloxide I verantwortlich.

Infolge der besonderen Halbwertsbreitenverhältnisse enthält das Pulver-Röntgenbeugungsspektrum der erfindungsgemäßen Multimetalloxide I die Beugungslinien $A^i$ nicht in vollständig voneinander aufgelöster Form. Das Vorhandensein der Mehrzahl der Beugungslinien $A^i$ zeigt sich jedoch (nach Abzug des linearen Untergrundes) in visuell unmittelbar erkenntlicher Weise als ein relatives Maximum in der Umrißlinie des Pulver-Röntgenbeugungsspektrums der neuen Multimetalloxide I, wobei die Lage dieser relativen Maxima in dieser Schrift die Lage der anspruchsgemäßen Beugungslinien $A^i$ definiert.

Nach Abzug des linearen Untergrundes (die Verbindungslinie zwischen A(2⊖ = 5°) und A(2⊖ = 65°)) lassen sich diesen Beugungslinien $A^1$ bis $A^{10}$ des weiteren die nachfolgenden, auf die intensitätsstärkste der Beugungslinien $A^1$ bis $A^{10}$ bezogenen, relativen Linienintensitäten $I_A^{rel} = (I/I_o) \times 100$ %) zuordnen, wobei als Intensitätsmaß die Amplitude, gemessen von der Grundlinie bis zum relativen Maximum, verwendet wird:

| Röntgenbeugungslinie | $I_A^{rel}$ [%] |
|---|---|
| $A^1$ | $7 \pm 5$ |
| $A^2$ | $5 \pm 5$ |
| $A^3$ | 100 |
| $A^4$ | $40 \pm 40$ |
| $A^5$ | $70 \pm 40$ |
| $A^6$ | $25 \pm 25$ |
| $A^7$ | $20 \pm 20$ |
| $A^8$ | $10 \pm 10$ |

(fortgesetzt)

| Röntgenbeugungslinie | $I_A^{rel}$ [%] |
|---|---|
| $A^9$ | $25 \pm 15$ |
| $A^{10}$ | $35 \pm 20$ |

Bezüglich dieser Angabe der Linienintensitäten sei vermerkt, daß die relativen Linienintensitäten, im Unterschied zur Lage der Linien, durch die sich bei verschiedenen Pulveraufnahmepräparationen, fußend auf der Anisotropie der Kristallform, einstellenden individuellen Kristallausrichtungen in dem Fachmann an sich bekannter Weise merklich beeinflußt werden und daher zur Identifikation der neuen Multimetalloxide eine geringere Signifikanz aufweisen.

In diesem Zusammenhang sei festgehalten, daß nach diesseitiger Beobachtung ein weiteres Charakteristikum des Cu-K$\alpha$-Pulver-Röntgenbeugungsspektrums der erfindungsgemäßen Multimetalloxide I darin zu liegen scheint, daß es in der Regel keine Beugungslinien enthält, deren FWHM < 0,25° beträgt.

In allen Ausführungsbeispielen erfolgte die Aufnahme des Pulver-Röntgenbeugungsspektrums mit einem Siemens Diffraktometer D-5000 unter Anwendung von Cu-K$\alpha$-Strahlung (40 kV, 30 mA, $\lambda$ = 1,54178 Å). Das Diffraktometer war mit einer automatischen Primär- und Sekundärstrahlblende sowie mit einem Graphit-Sekundärmonochromator ausgestattet.

Selbstverständlich läßt sich die Auflösung, und damit eine alternative Charakterisierung des Pulver-Röntgenbeugungsspektrums der Multimetalloxide I, dadurch erzeugen, daß man nach Abzug des linearen Untergrundes unter der Randbedingung einer minimalen mittleren quadratischen Abweichung das experimentelle Beugungsspektrum (seine Umrißlinie) durch eine mathematische Funktion beschreibt und durch eine Entfaltung in eine Superposition von separat aufgelösten Linien $A^{1*}$ bis $A^{10*}$ überführt. Das Maximum der aufgelösten Linien definiert jeweils deren Linienlage.

Die wie vorstehend beschriebene mathematische Auflösung der Pulver-Röntgenbeugungsspektren erfolgte mit dem von der Firma Siemens bereitgestellten Software-Paket DIFFRAC-AT V 3.2/PROFILE in der Version von 1994, wobei die Entfaltung und Anpassung auf der Basis der Pearson-VII-Gestaltsfunktion vorgenommen wurde.

Während das Vorhandensein der Beugungslinien $A^2$, $A^4$ und $A^6$ bis $A^8$ in den Cu-K$\alpha$-Pulver-Röntgenbeugungsspektren der erfindungsgemäßen Multimetalloxide I auf der Grundlage einer ausschließlich visuellen Betrachtung ihrer Umrißlinie teilweise mit einer gewissen Unsicherheit behaftet und dadurch gegebenenfalls strittig ist (vgl. Bsp.), weist ihre wie ebenda beschrieben erzeugte mathematische Auflösung in der Regel in zweifelsfreier Weise Beugungslinien $A^{2*}$, $A^{4*}$, $A^{6*}$ und $A^{7*}$ aus (vgl. Bsp.), wohingegen sie einer Beugungslinie $A^{8*}$ nicht bedarf. Beide Charakterisierungsarten (Informationen) zusammen machen daher die anspruchsgemäße Definition des erfindungsgemäßen Gegenstandes erforderlich. Ausschließlich mathematisch aufgelöst läßt sich das Cu-K$\alpha$-Pulver-Röntgenbeugungsspektrum der erfindungsgemäßen Multimetalloxide I in der Regel hingegen wie folgt charakterisieren:

| Röntgenbeugungslinie | $2\ominus$ [°] | $I_F^{rel}$ [%] | FWHM [°] |
|---|---|---|---|
| $A^{1*}$ | $8,1 \pm 0,7$ | $8 \pm 7$ | $3,0 \pm 0,8$ |
| $A^{2*}$ | $14,0 \pm 0,8$ | $6 \pm 5$ | $2,4 \pm 1,5$ |
| $A^{3*}$ | $22,2 \pm 0,2$ | $30 \pm 20$ | $1,2 \pm 0,9$ |
| $A^{4*}$ | $23,2 \pm 0,7$ | $23 \pm 20$ | $1,8 \pm 1,2$ |
| $A^{5*}$ | $27,0 \pm 0,5$ | $80 \pm 20$ | $4,0 \pm 2,5$ |
| $A^{6*}$ | $31,8 \pm 1,5$ | $52 \pm 48$ | $5,0 \pm 3,0$ |
| $A^{7*}$ | $34,7 \pm 1,2$ | $52 \pm 48$ | $4,0 \pm 3,0$ |
| $A^{8*}$ | - | - | - |
| $A^{9*}$ | $45,4 \pm 0,4$ | $12 \pm 8$ | $1,5 \pm 1,0$ |
| $A^{10*}$ | $48,6 \pm 0,5$ | $25 \pm 20$ | $2,2 \pm 1,8$ |

$I_F^{rel}$ [%] ist dabei die auf die intensitätsstärkste der Beugungslinien $A^{1*}$ bis $A^{10*}$ bezogene prozentuale Linienintensität, wobei als Intensitätsmaß die Fläche unter der Beugungslinie verwendet wird.

Die erfindungsgemäßen Multimetalloxide I sind in einfacher Weise dadurch erhältlich, daß man von geeigneten Quellen ihrer elementaren Konstituenten ein möglichst inniges, vorzugsweise feinteiliges, Trockengemisch erzeugt, das der gewünschten Stöchiometrie entspricht, und dieses bei Temperaturen im Bereich von 300 bis 500°C calciniert. Den Schlüssel zum Zugang zu den erfindungsgemäßen Multimetalloxiden I bildet dabei der Redoxcharakter der Calcinationsatmosphäre, der nicht zu oxidierend und nicht zu reduzierend sein darf.

Allgemein gültige quantitative Angaben bezüglich des erforderlichen Redoxcharakters der Calcinationsatmosphäre sind nicht möglich. So variiert der erforderliche Redoxcharakter zum einen mit der gewählten Stöchiometrie und zum anderen tragen beispielsweise auch die Gegenionen (z.B. $NH_4^\oplus$) der gewählten Quellen der Elementkonstituenten, z.B. bei ihrer Zersetzung während des Calcinierens, zum Redoxcharakter der Calcinationsatmosphäre bei. Durch orientierende Vorversuche kann der Fachmann den erforderlichen Redoxcharakter der Calcinationsatmosphäre jedoch ermitteln.

Zur Steuerung des Redoxcharakters der Calcinationsatmosphäre stehen dem Fachmann dabei als Inertgase z.B. $N_2$ und/oder Edelgase, als reduzierende Gase z.B. Ammoniak, Wasserstoff, niedermolekulare Aldehyde und/oder Kohlenwasserstoffe und als oxidatives Gas Sauerstoff (meist in Form von Luft) zur Verfügung.

Wesentlich für die Elementquellen ist nur, daß es sich entweder bereits um Oxide handelt oder um solche Verbindungen, die durch Erhitzen, wenigstens im Beisein von Sauerstoff, in Oxide überführbar sind. Neben den Oxiden kommen daher als Ausgangsverbindungen vor allem Halogenide, Nitrate, Formiate, Citrate, Oxalate, Acetate, Carbonate oder Hydroxide in Betracht. Geeignete Ausgangsverbindungen des Mo, V, W und Nb sind auch deren Oxoverbindungen (Molybdate, Vanadate, Wolframate und Niobate), die in der Regel $NH_4^\oplus$ als Gegenion aufweisen, welches beim Erhitzen reduktiv wirkenden NH3 freisetzt. Selbstverständlich kommen auch die von diesen Oxoverbindungen abgeleiteten Säuren als mögliche Ausgangsverbindungen in Betracht. Zusätzlich werden den Elementquellen häufig Verbindungen wie Ammoniumacetat und Ammoniumnitrat zugesetzt, die während der Calcination als Porenbildner fungieren und gleichfalls den Redoxcharakter der Calcinationsatmosphäre beeinflußen.

Das innige Vermischen der Ausgangsverbindungen im Rahmen der Herstellung von Multimetalloxiden I kann in trockener oder in nasser Form erfolgen. Erfolgt es in trockener Form, werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden die Ausgangsverbindungen dabei in Form einer wäßrigen Lösung und/oder Suspension miteinander vermischt. Anschließend wird die wäßrige Masse getrocknet und nach Trocknung calciniert. Vorzugsweise erfolgt der Trocknungsprozeß unmittelbar im Anschluß an die Fertigstellung der wäßrigen Mischung und durch Sprühtrocknung (die Eintrittstemperatur beträgt in der Regel 250 bis 350°C und die Austrittstemperaturen liegen normalerweise bei 100 bis 150°C).

Interessanterweise eignen sich die neuen Multimetalloxide I bereits für sich als Aktivmasse von Katalysatoren für die gasphasenkatalytische Oxidation von organischen Verbindungen wie 3 bis 6 C-Atome aufweisender Alkane, Alkanole, Alkene, Alkanale, Alkenale und Alkenole (z.B. Propylen, Methacrolein, tert.-Butanol, Methylester des tert.-Butanol, iso-Buten, iso-Butan oder iso-Butyraldehyd) zu olefinisch ungesättigten Aldehyden und/oder Carbonsäuren, sowie den entsprechenden Nitrilen (Ammoxidation, vor allem von Propen zu Acrylnitril und von iso-Buten bzw. tert.-Butanol zu Methacrylnitril). Beispielhaft genannt sei die Herstellung von Acrolein, Methacrolein und Methacrylsäure. Ferner eignen sie sich auch als Katalysatoraktivmassen zur oxidativen Dehydrierung olefinischer Verbindungen.

In besonderer Weise eignen sich die Multimetalloxide I dabei zweifellos als Aktivmasse von Katalysatoren für die gasphasenkatalytische Oxidation von Acrolein zu Acrylsäure, wobei bezüglich dieser Umsetzung insbesondere ihre erhöhte Aktivität besticht.

Bei Verwendung der erfindungsgemäßen Multimetalloxidmassen als Aktivmasse von Katalysatoren für die gasphasenkatalytische Oxidation organischer Verbindungen, insbesondere Acrolein zu Acrylsäure, erfolgt die Formgebung zur gewünschten Katalysatorgeometrie vorzugsweise durch Aufbringen auf vorgeformte inerte Katalysatorträger, wobei das Aufbringen vor oder nach der abschließenden Calcination erfolgen kann. Dabei können die üblichen Trägermaterialien wie poröse oder unporöse Aluminiumoxide, Siliciumdioxid, Thoriumdioxid, Zirkondioxid, Siliciumcarbid oder Silicate wie Magnesium- oder Aluminiumsilicat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Unter diesen sind wiederum Kugeln besonders vorteilhaft. Von besonderem Vorteil ist die Verwendung von im wesentlichen unporösen, oberflächenrauhen, kugelförmigen Trägern aus Steatit, deren Durchmesser 1 bis 6 mm, bevorzugt 4 bis 5 mm beträgt. Die Schichtdicke der Aktivmasse wird zweckmäßigerweise als im Bereich 50 bis 500 µm, bevorzugt im Bereich 150 bis 250 µm liegend gewählt. Es sei an dieser Stelle darauf hingewiesen, daß bei der Herstellung solcher Schalenkatalysatoren zur Beschichtung der Trägerkörper die aufzubringende Pulvermasse in der Regel befeuchtet und nach dem Aufbringen, z.B. mittels heißer Luft, wieder getrocknet wird.

Die Beschichtung der Trägerkörper wird zur Herstellung der Schalenkatalysatoren in der Regel in einem geeigneten drehbaren Behälter ausgeführt, wie er z.B. aus der DE-A 2 909 671 oder aus der EP-A 293 859 vorbekannt ist. In der Regel wird die relevante Masse vor der Trägerbeschichtung calciniert. Ferner wird das katalytisch aktive Material vor Durchführung der Beschichtung auf einen Teilchendurchmesser im Bereich von > 0 bis 300 µm, bevorzugt 0,1 bis 200 µm, besonders bevorzugt 0,5 bis 50 µm, gemahlen. In geeigneter Weise kann das Beschichtungs- und Calcinierungsverfahren auch gemäß der EP-A 293 859 so durchgeführt werden, daß die resultierenden Multimetalloxid-I-Schichten eine spezifische Oberfläche von 0,50 bis 150 m$^2$/g, ein spezifisches Porenvolumen von 0,10 bis 0,90 cm$^3$/g und eine solche Porendurchmesser-Verteilung aufweisen, daß auf die Durchmesserbereiche 0,1 bis < 1 µm, 1,0 bis < 10 µm und 10 µm bis 100 µm jeweils wenigstens 10 % des Porengesamtvolumens entfallen. Auch können die in der EP-A 293 859 als bevorzugt genannten Porendurchmesser-Verteilungen eingestellt werden.

Sebstverständlich können die erfindungsgemäßen Multimetalloxide I auch als Vollkatalysatoren betrieben werden. Diesbezüglich wird das die Ausgangsverbindungen des Multimetalloxids I umfassende innige Trockengemisch vorzugsweise unmittelbar zur gewünschten Katalysatorgeometrie verdichtet (z.B. Tablettieren, Extrudieren oder Strangpressen), wobei gegebenenfalls an sich übliche Hilfsmittel, z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können, und calciniert. Generell kann auch hier vor der Formgebung calciniert werden.

Bevorzugte Vollkatalysatorgeometrie sind Hohlzylinder mit einem Außendurchmesser und einer Länge von 2 bis 10 mm und einer Wandstärke von 1 bis 3 mm.

Zur katalytischen Gasphasenoxidation von Acrolein zu Acrylsäure mit den neuen Multimetalloxiden I als Katalysatoraktivmasse, wird normalerweise Acrolein eingesetzt, das durch die katalytische Gasphasenoxidation von Propen erzeugt wurde. In der Regel werden die Acrolein enthaltenden Reaktionsgase aus dieser Propenoxidation ohne Zwischenreinigung eingesetzt. Üblicherweise wird die gasphasenkatalytische Oxidation des Acroleins in Rohrbündelreaktoren als heterogene Festbettoxidation ausgeführt. Als Oxidationsmittel wird in an sich bekannter Weise Sauerstoff, zweckmäßigerweise mit inerten Gasen verdünnt (z.B. in Form von Luft), eingesetzt. Geeignete Verdünner sind z.B. $N_2$, $CO_2$, Kohlenwasserstoffe wie Methan, Ethan, Propan, Butan und/oder Pentan, rückgeführte Reaktionsabgase und/oder Wasserdampf. In der Regel wird bei der Acroleinoxidation ein Acrolein : Sauerstoff : Wasserdampf : Inertgas-Volumenverhältnis von 1 : (1 bis 3) : (0 bis 20) : (3 bis 30), vorzugsweise von 1 : (1 bis 3) : (0,5 bis 10) : (7 bis 18) eingestellt. Der Reaktionsdruck beträgt im allgemeinen 1 bis 3 bar und die Gesamtraumbelastung beträgt vorzugsweise 1000 bis 3500 Nl/l/h. Typische Vielrohr-Festbettreaktoren sind z.B. in den Schriften DE-A 2 830 765, DE-A 2 201 528 oder US-A 3 147 084 beschrieben. Die Reaktionstemperatur wird üblicherweise so gewählt, daß der Acroleinumsatz bei einfachem Durchgang oberhalb von 90 %, vorzugsweise oberhalb von 98 %, liegt. Im Normalfall sind diesbezüglich Reaktionstemperaturen von 230 bis 330°C erforderlich.

Bemerkenswerterweise weisen die erfindungsgemäßen Multimetalloxide I im Rahmen der gasphasenkatalytischen Oxidation von Acrolein zu Acrylsäure nicht nur eine erhöhte Aktivität, sondern auch eine erstaunlich geringe Formierungszeit auf; d.h., wird ein mit den erfindungsgemäßen Multimetalloxiden I beschickter Rohrbündel reaktor unter den vorgenannten Bedingungen mit einem Acrolein enthaltenden Gasstrom zum Zweck der oxidativen Bildung von Acrylsäure betrieben, so erreicht die Selektivität der Acrylsäurebildung bereits innerhalb einer reduzierten Betriebsdauer ihren Plateauwert. Bezüglich dieses Plateauwertes verfügt die Herstellung der erfindungsgemäßen Multimetalloxidmassen über eine erhöhte Reproduzierbarkeit.

Ihr Potential als Katalysatoraktivmasse für die katalytische Gasphasenoxidation organischer Verbindungen, insbesondere von Acrolein zu Acrylsäure, beginnen die Multimetalloxide I unter Beibehalt der vorgenannten Vorteile jedoch erst dann in vollem Umfang zu entfalten, wenn man sie mit Multimetalloxiden der allgemeinen Formel II

$$M^3_{12} \, Cu_d \, H_e \, O_y \qquad\qquad (II),$$

mit

$M^3 =$   Mo, W, V, Nb und/oder Ta,

$d =$   4 bis 30, vorzugsweise 6 bis 24, besonders bevorzugt 9 bis 17,

$e =$   0 bis 20, vorzugsweise 0 bis 10 und

$y =$   eine Zahl, die durch die Wertigkeit und Häufigkeit der von Sauerstoff verschiedenen Elemente in II bestimmt wird,

verdünnt und so wenigstens zweiphasige Multimetalloxidmassen der allgemeinen Formel III

$$[A]_p \, [B]_q \qquad\qquad (III),$$

in der die Variablen folgende Bedeutung haben:

$$A = (Mo_{12-a-b-c} \, V_a \, M^1_b \, M^2_c \, O_x) \times \frac{12}{12\text{-}a\text{-}b\text{-}c} \qquad \text{(Aktivphase),}$$

$$B = M^3_{12} \, Cu_d \, H_e \, O_y \qquad \text{(Promotorphase),}$$

p,q =  von Null verschiedene Zahlen, deren Verhältnis p/q 160:1 bis 1:1, vorzugsweise 20:1 bis 1:1 und besonders bevorzugt 15:1 bis 4:1 beträgt,

erzeugt, die den Anteil $[A]_p$ in Form dreidimensional ausgedehnter, von ihrer lokalen Umgebung aufgrund ihrer von ihrer lokalen Umgebung verschiedenen chemischen Zusammensetzung abgegrenzter, Bereiche A der chemischen Zusammensetzung

$$A \qquad Mo_{12-a-b-c} \, V_a \, M^1_b \, M^2_c \, O_x$$

und den Anteil [B], in Form dreidimensional ausgedehnter, von ihrer lokalen Umgebung aufgrund ihrer von ihrer lokalen Umgebung verschiedenen chemischen Zusammensetzung abgegrenzter, Bereiche B der chemischen Zusammensetzung

$$B \qquad M^3_{12} \, Cu_d \, H_e \, O_y$$

enthalten, wobei die Bereiche A, B relativ zueinander wie in einem Gemisch aus feinteiligem A und feinteiligem B verteilt sind.

In einfachster Weise bildet man zur Herstellung solcher Multimetalloxidmassen III die Multimetalloxide I und II jeweils getrennt vor und erzeugt dann aus wenigstens einem feinteiligen, getrennt vorgebildeten, Multimetalloxid I und wenigstens einem feinteiligen, getrennt vorgebildeten, Multimetalloxid II im gewünschten Mengenverhältnis ein inniges Trockengemisch (das innige Vermischen kann dabei in trockener oder in nasser Form erfolgen; erfolgt es in nasser, in der Regel wäßriger, Form, wird anschließend getrocknet; das Vermischen kann durch Kneter oder Mischer bewirkt werden), aus welchem dann, in entsprechender Weise wie für das Multimetalloxid I alleine beschrieben, durch Formgebung geeignete Katalysatoren für die katalytische Gasphasenoxidation organischer Verbindungen, insbesondere jene von Acrolein zu Acrylsäure, erzeugt werden können. Selbstverständlich können aber auch sowohl die Multimetalloxide I als auch die Multimetalloxidmassen III in Pulverform als Katalysatoren eingesetzt werden. Ebenso selbstverständlich können die Multimetalloxidmassen III für all jene katalytischen Gasphasenreaktionen eingesetzt werden, für die auch die Multimetalloxide I als geeignet genannt wurden. Werden Katalysatoren auf der Basis von Multimetalloxidmassen III als Aktivmasse für die katalytische Gasphasenoxidation von Acrolein zu Acrylsäure angewendet, so erfolgt dies in der Regel unter denselben Verfahrensbedingungen wie für die entsprechende alleinige Anwendung der Multimetalloxid-I-Katalysatoren beschrieben.

Die Multimetalloxide II können in einfacher, dem Fachmann an sich bekannter Weise z.B. dadurch hergestellt werden, daß man von geeigneten Quellen ihrer elementaren Konstituenten ein möglichst inniges, vorzugsweise feinteiliges, Trockengemisch erzeugt und dieses bei Temperaturen von 200 bis 1000°C, häufig 250 bis 600°C, vielfach 300 bis 500°C calciniert, wobei die Calcination unter Inertgas (z.B. $N_2$), einem Gemisch aus Inertgas und Sauerstoff (z.B. Luft), reduzierend wirkenden Gasen wie Kohlenwasserstoffen (z.B. Methan), Aldehyden (z.B. Acrolein) oder Ammoniak, aber auch unter einem Gemisch aus $O_2$ und reduzierend wirkenden Gasen (z.B. allen vorgenannten) erfolgen kann, wie es beispielsweise in der DE-A 4 335 973 beschrieben wird. Bemerkenswerterweise kann die Calcinationsatmosphäre hier auch Wasserdampf umfassen. Bei einer Calcination unter reduzierenden Bedingungen ist zu beachten, daß die metallischen Konstituenten nicht bis zum Element reduziert werden. Mit zunehmender Calcinierungstemperatur nimmt die Calcinationsdauer in der Regel ab.

Hinsichtlich der Quellen der elementaren Konstituenten der Multimetalloxide II gilt im wesentlichen das gleiche wie für die Quellen der elementaren Konstituenten der Multimetalloxide I. Wesentlich ist auch hier nur, daß es sich entweder bereits um Oxide handelt oder um solche Verbindungen, die durch Erhitzen, wenigstens in Anwesenheit von Sauerstoff, in Oxide überführbar sind. In einer bevorzugten Herstellvariante der Multimetalloxide II erfolgt die thermische Behandlung des innigen Gemisches der verwendeten Ausgangsverbindungen in einem Autoklaven im Beisein von überatmosphärischen Druck aufweisendem Wasserdampf bei Temperaturen von > 100 bis 600°C.

Je nach den gewählten Calcinationsbedingungen weisen die resultierenden Multimetalloxide II verschiedene atomare Raumanordnungen auf. Insbesondere kommen für die vorliegende Erfindung all jene Multimetalloxide II in Betracht, die in der DE-A 4 405 514, der DE-A 4 440 891 und der DE-A 19 528 646 als mögliche Schlüsselphase bzw.

Promotorphase beschrieben sind. D.h., günstige Multimetalloxide II sind u.a. jene, die den Strukturtyp (das Röntgenbeugungsmuster) wenigstens eines der in der nachfolgenden Tabelle 1 aufgelisteten Kupfermolybdate aufweisen (der Ausdruck in Klammern gibt die Quelle für den zugehörigen Röntgenbeugungsfingerabdruck wieder):

| $Cu_3(MoO_4)_2(OH)_2$ | (Lindgrenit, Karteikarte 36-405 der JCPDS-ICDD Kartei (1991)), |
|---|---|
| $Cu_4MoO_6O_{20}$ | (A. Moini et al., Inorg. Chem. 25(21) (1986) S. 3782 bis 3785), |
| $Cu_4Mo_5O_{17}$ | (Karteikarte 39-181 der JCPDS-ICDD Kartei (1991)), |
| $Cu_6Mo_5O_{18}$ | (Karteikarte 40-865 der JCPDS-ICDD Kartei (1991)), |
| $Cu_6Mo_4O_{15}$ | (Karteikarte 35-17 der JCPDS-ICDD Kartei (1991)), |
| $CuMoO_4$ | (Karteikarte 22-242 der JCPDS-ICDD Kartei (1991)), |
| $CuMoO_4$ | (Russian Journal of Inorganic Chemistry 36(7), 1991, S. 927-928, Table 1, $CuMoO_4$-III mit verzerrter Wolframit-Struktur ($CuWO_4$, Karteikarte 21-307 JCPDS-ICDD Kartei (1994)), |
| $CuMoO_4$ | (Karteikarte 26-546 der JCPDS-ICDD Kartei (1991)), |
| HT-Cu-Molybdat | (DE-A 19 528 646), |
| $Cu_{4-X}Mo_3O_{12}$ | mit x = 0 bis 0,25 (Karteikarte 24-56 und 26-547 der JCPDS-ICDD Kartei (1991)), |
| $Cu_3Mo_2O_9$ | (Karteikarte 24-55 und 34-637 der JCPDS-ICDD Kartei (1991)) und |
| $Cu_2MoO_5$ | (Karteikarte 22-607 der JCPDS-ICDD Kartei (1991)). |

Als zur Herstellung von für die katalytische Gasphasenoxidation von Acrolein zu Acrylsäure besonders geeigneten Multimetalloxidmassen III zu empfehlende Multimetalloxide II sind solche der allgemeinen Formel IV

$$CuMo_AW_BV_CNb_DTa_EO_Y \cdot (H_2O)_F \qquad \text{(IV)},$$

mit

$1/(A+B+C+D+E)$ = 0,7 bis 1,3,
$F$ = 0 bis 1,
$B+C+D+E$ = 0 bis 1 und
$Y$ = eine Zahl, die durch die Wertigkeit und Häufigkeit der von Sauerstoff verschiedenen Elemente in IV bestimmt wird,

deren atomare Raumanordnung dem Strukturtyp entspricht, der durch die Verbindung $CuMoO_4$-III in Russian Journal of Inorganic Chemistry 36 (7), 1991 auf S. 921 in Table 1 definiert wird und in der DE-A 4 405 514 sowie der DE-A 4 440 891 als Wolframit bezeichnet wird, zu nennen.

Unter den Multimetalloxiden IV sind wiederum jene der Stöchiometrie V

$$CUMO_AW_BV_CO_Y \qquad \text{(V)},$$

mit

$1/(A+B+C)$ = 0,7 bis 1,3,
A, B, C = alle > 0, mit der Maßgabe, daß $B+C \leq 1$,
$Y$ = eine Zahl, die durch die Wertigkeit und Häufigkeit der von Sauerstoff verschiedenen Elemente in V bestimmt wird,

sowie jene der Stöchiometrie VI

$$CuMO_AW_BO_y \qquad \text{(VI)},$$

mit

$1/(A+B)$ = 0,7 bis 1,3,

$B/A$ = 0,01 bis 10, vorzugsweise 0,01 bis 1, und

$Y$ = eine Zahl, die durch die Wertigkeit und Häufigkeit der von Sauerstoff verschiedenen Elemente in VI bestimmt wird,

hervorzuheben. Die Herstellung von Multimetalloxiden IV, V und VI offenbart die DE-A 4 405 514 und die DE-A 4 440 891.

Als weitere besonders geeignete Multimetalloxide II sind jene der allgemeinen Formel VII zu nennen,

$$CuMo_A'W_B'V_C'Nb_D'Ta_E'O_Y' \qquad\qquad (VII),$$

mit

$1/(A'+B'+C'+D'+E')$ = 0,7 bis 1,3, vorzugsweise 0,85 bis 1,15, besonders bevorzugt 0,95 bis 1,05 und ganz besonders bevorzugt 1,

$(B'+C'+D'+E')/A'$ = 0,01 bis 10, vorzugsweise 0,05 bis 3 und besonders bevorzugt 0,075 bis 1,5 und

$Y'$ = eine Zahl, die durch die Wertigkeit und Häufigkeit der von Sauerstoff verschiedenen Elemente in VII bestimmt wird,

deren Strukturtyp der in der DE-A 19 528 646 offenbarte HT-Cu-Molybdat-Typ ist.

Unter den Multimetalloxiden VII sind dabei wiederum jene mit C'+D'+E'= O bevorzugt. Ein als Multimetalloxid II besonders empfehlenswertes VII ist jenes der Zusammensetzung $Mo_{10,8}W_{1,2}Cu_{12}O_{42-48}$.

Ferner sind als mögliche Multimetalloxide II Mischungen aus Multimetalloxiden IV und VII zu nennen, wie sie die DE-A 19 528 646 ebenfalls offenbart. Dies gilt insbesondere für jene Gemische, die die Multimetalloxide IV und VII in miteinander verwachsener Form enthalten.

Selbstverständlich kann das aus getrennt vorgebildeten feinteiligen Multimetalloxiden I und II erzeugte Gemisch vor seiner Verwendung zur Herstellung von Multimetalloxidmassen-III-Katalysatoren auch zunächst noch verpreßt, danach gemahlen und dann erst in die gewünschte Katalystorform gebracht werden. Für den Korngrößtdurchmesser wird dabei, ebenso wie im Fall der getrennt vorgebildeten Multimetalloxide I und II, der Durchmesserbereich von > 0 bis 300 µm, besonders bevorzugt 0,5 bis 50 µm und ganz besonders bevorzugt 1 bis 30 µm empfohlen.

Der besondere Vorteil der erfindungsgemäßen mehrphasigen Multimetalloxidmassen-III-Katalystoren liegt darin begründet, daß die zum Erhalt der verschiedenen Phasen erforderlichen Calcinationsbedingungen in der Regel voneinander verschieden sind. Durch das Prinzip der getrennten Vorbildung können die Calcinationsbedingungen der jeweils gewünschten Phase optimal angepaßt werden.

Die Multimetalloxide I eignen sich über die vorgenannten Verwendungen hinaus ganz generell zur Verbesserung der Performance von katalytisch aktiven Multimetalloxidmassen wie sie z.B. in der DE-A 4 335 973, US-A 4 035 262, DE-A 4 405 058, DE-A 4 405 059, DE-A 4 405 060, DE-A 4 405 514, DE-A 4 440 891 und DE-A 19 528 646 beschrieben sind.

In einfacher Weise werden dazu z.B. die dort beschriebenen Aktivmassen in feinteiliger Form mit feinteiligen Multimetalloxiden I gemischt und wie das innige Ausgangsgemisch zur Herstellung von Multimetalloxidmassen-III-Katalysatoren geformt.

Insbesondere eignen sich die Multimetalloxide I dabei als Bestandteil der Co-Phasen der in den DE-A 4 405 058, DE-A 4 405 059, DE-A 4 405 060, DE-A 4 405 514 und DE-A 4 440 891 offenbarten mehrphasigen Multimetalloxidmassenkatalysatoren. Ferner eignen sich die Multimetalloxide I als Bestandteil der Aktivphase der mehrphasigen Multimetalloxidmassenkatalysatoren der DE-A 19 528 646. In all diesen Fällen entstehen mehrphasige Multimetalloxidmassenkatalysatoren, die Phasen aus Multimetalloxiden I in feinteiliger (> 0 bis 300 µm) homogener Verteilung enthalten. Sie eignen sich insbesondere als Katalysatoren für die in den jeweiligen zitierten Schriften genannten katalytischen Gasphasenoxidationen organischer Verbindungen.

Abschließend sei festgehalten, daß die vorliegende Erfindung erstmals die Reindarstellung von Multimetalloxiden I ermöglicht.

Beispiele

1) Herstellung und Identifizierung von erfindungsgemäßen Multimetalloxiden MI1 bis MI9 und Vergleichsmultimetalloxiden VMI1 und VMI2

Allgemeines

Die nachfolgend beschriebenen erfindungsgemäßen Multimetalloxide I enthalten die elementaren Konstituenten Mo und W stets im Oxidationszustand +6. Der Oxidationszustand des elementaren Konstituenten V ist dagegen in der Regel eine Verteilung auf dessen mögliche Oxidationszustände $V^{3+}$, $V^{4+}$ und $V^{5+}$. Die Bestimmung dieser Verteilung war durch titrimetrische Maßanalyse mit potentiometrischer Endpunktanzeige (kombinierte Platinelektrode und Potentiograph der Fa. Metrohm, 9100 Herisau, Schweiz, CH) möglich, wobei unter Inertgasatmosphäre gearbeitet werden mußte. Die Durchführung der Titration bei 80°C erleichterte die Endpunktserkennung. Im übrigen gestaltete sich die potentiometrische Maßanalyse in allen Fällen wie folgt:

Jeweils 0,15 g des oxidischen Probenmaterials wurde in einem Gemisch aus 5 ml konzentrierter Phosphorsäure (Massendichte $\varrho$ bei 20°C = 1,70 g/cm$^3$) und 10 ml wäßriger Schwefelsäure unter Erwärmen aufgelöst (Argonatmosphäre), wobei die verwendete wäßrige Schwefelsäurelösung ihrerseits ein Gemisch aus gleichen Volumina Wasser und konzentrierter Schwefelsäure ($\varrho^{20}$ = 1,52 g/cm$^3$) war (die Volumenangaben beziehen sich auf 20°C). Das Lösungsmittel war so gewählt, daß es die möglichen Oxidationszustände des V nicht veränderte, was mittels entsprechender V-Standards überprüft wurde.

Zur analytischen Bestimmung des $V^{5+}$-Gehaltes wurde die frisch zubereitete resultierende Lösung mit einer 0,1 molaren wäßrigen Ammoniumeisensulfat-Maßlösung ($(NH_4)_2Fe(SO_4)_2$) titriert. Zur analytischen Bestimmung von $V^{3+}$ und $V^{4+}$ wurde eine in entsprechender Weise zubereitete frische Lösung mit einer frisch hergestellten 0,02 molaren wäßrigen Kaliumpermanganat-Maßlösung ($KMnO_4$) titriert, wobei zwei Potentialsprünge auftraten ($V^{3+} \rightarrow V^{4+}$ und $V^{4+} \rightarrow V^{5+}$). Die Summe der wie beschrieben ermittelten $V^{3+}$-, $V^{4+}$- und $V^{5+}$-Gehalte mußte dem V-Gesamtgehalt der Probe entsprechen. Dieser ließ sich ebenfalls durch titrimetrische Maßanalyse mit potentiometrischer Endpunktanzeige ermitteln.

Dazu wurden 0,15 g des oxidischen Probenmaterials in einem Gemisch aus 10 ml der vorgenannten halbkonzentrierten wäßrigen Schwefelsäure und 10 ml konzentrierter Salpetersäure ($\varrho^{20}$ = 1,52 g/cm$^3$) unter Erwärmen aufgelöst (Argonatmosphäre). Anschließend wurde die erhaltene Lösung durch Erhitzen unter Abrauchen der Salpetersäure und eines Teils der eingesetzten Schwefelsäure auf ein Restvolumen von ca. 3 ml eingeengt, wobei der gesamte V-Anteil in die Oxidationsstufe +5 überführt wurde. Nach Abkühlen wurde das Restvolumen auf 50 ml verdünnt und das enthaltene $V^{5+}$ mit einer 0,1 molaren wäßrigen $(NH_4)_2Fe(SO_4)_2$-Maßlösung über den Äquivalenzpunkt (Endpunkt) hinaus potentiometrisch titriert.

Die so erhaltene wäßrige Lösung wurde mit einer frisch hergestellten 0,02 molaren wäßrigen Kaliumpermanganat-Maßlösung titriert, wobei zwei Potentialsprünge auftraten. Der erste Potentialsprung indizierte den eingesetzten Überschuß an $Fe^{2+}$, der zweite Potentialsprung lieferte die zur Oxidation von $V^{4+}$ zu $V^{5+}$ benötigte Menge an $KMnO_4$, die dem V-Gesamtgehalt der Probe entspricht.

$$MI1: \quad Mo_{8,54}V_{2,47}W_{0,99}O_{33,53}$$

33,746 kg Ammoniumheptamolybdathydrat ($MoO_3$-Gehalt: 81,8 Gew.-%, Idealzusammensetzung: $(NH_4)_6Mo_7O_{24}$ x $4H_2O$), 6,576 kg Ammoniummetavanadat ($V_2O_5$-Gehalt: 76,5 Gew.-%, Idealzusammensetzung: $NH_4VO_3$), 5,764 kg Ammoniumparawolframathydrat ($WO_3$-Gehalt: 89,0 Gew.-%, Idealzusammensetzung: $(NH_4)_{10}W_{12}O_{41}$ x $7H_2O$) und 7,033 kg Ammoniumacetat ($CH_3COONH_4$-Gehalt: 97,0 Gew.-%, Idealzusammensetzung: $CH_3COONH_4$) wurden in der genannten Reihenfolge nacheinander bei einer Temperatur von 90°C in 250 l Wasser unter Rühren gelöst. Die resultierende gelb- bis orangefarbene Lösung wurde auf 80°C abgekühlt und mit einer Eingangstemperatur von 300°C und einer Ausgangstemperatur von 110°C sprühgetrocknet.

800 g des erhaltenen Sprühpulvers wurden in einem Kneter (Typ LUK 2,5 der Fa. Werner und Pfleiderer, 7000 Stuttgart, DE) mit einem Nutzvolumen von 2,5 l unter Zusatz von 250 g Wasser 1 h geknetet. Dabei wurden von den 250 g Wasser 180 g innerhalb der ersten 10 Minuten der Knetung und 70 g innerhalb der restlichen 50 Minuten der Knetung zugesetzt. Das resultierende feuchte und schollenförmige Knetprodukt wurde 15 h bei einer Temperatur von 110°C getrocknet und danach durch ein Sieb mit einer Maschenweite von 5 mm gedrückt.

100 g des dabei resultierenden Granulates wurden anschließend in einem waagerechten Drehkugelofen mit einem isotherm beheizten Quarzkugelvolumen von 1 l und einer Drehgeschwindigkeit von 12 Umdrehungen pro Minute calciniert. Die Calcinationsbedingungen gestalteten sich dabei wie folgt:

| 1. | Schritt | innerhalb von 50 min das eingesetzte Granulat kontinuierlich von 25 auf 275°C erhitzen; |
|---|---|---|
| 2. | Schritt | innerhalb von 30 min das eingesetzte Granulat kontinuierlich von 275°C auf 325°C erhitzen; |
| 3. | Schritt | das eingesetzte Granulat 4 h bei 325°C halten; |
| 4. | Schritt | innerhalb von 30 min das eingesetzte Granulat kontinuierlich von 325°C auf 400°C erhitzen; |
| 5. | Schritt | das eingesetzte Granulat 10 min bei 400°C halten; |

Anschließend wurde die äußere Beheizung des Drehkugelofens abgeschaltet und letztere durch äußeres Anblasen mit Umgebungsluft gekühlt. Dabei kühlte sich das eingesetzte Granulat innerhalb von 5 h auf 25°C ab.

Während der einzelnen Calcinationsschritte wurde der Innenraum des Drehkugelofens parallel zur Drehachse von Gasmischungen durchströmt, die nachfolgende Zusammensetzungen aufwiesen (Normalbedingungen (N) = 1 atm, 25°C):

1. Schritt, 2. Schritt und 3. Schritt:
3,6 Nl/h Luft, 1,5 Nl/h $NH_3$ und 44,9 Nl/h $N_2$
(Gesamtgasfluß: 50 Nl/h);

4. Schritt, 5. Schritt und Abkühlphase:
3,6 Nl/h Luft und 44,9 Nl/h $N_2$
(Gesamtgasfluß: 48,5 Nl/h).

Das resultierende pulverförmige Multimetalloxid MI1 wies eine schwarze Farbe und eine spezifische Oberfläche (bestimmt nach DIN 66 131 durch Gasadsorption ($N_2$) gemäß Brunauer-Emmet-Teller (BET)) von 15,0 $m^2/g$ auf. Das im Multimetalloxid MI1 enthaltene Vanadium lag zu mehr als 99 % als $V^{4+}$ vor (die Ermittlung erfolgte wie vorstehend beschrieben). Damit wies das Multimetalloxid MI1 die Stöchiometrie $Mo_{8,54}V_{2,47}W_{0,99}O_{33,53}$ auf.

Das gemäß den Ausführungen in der Beschreibung ermittelte und nach Abzug des linearen Untergrundes ausgewertete Cu-K$\alpha$-Pulver-Röntgenbeugungsspektrum enthielt im relevanten 2$\ominus$-Bereich (5° bis 50°) die nachfolgenden Beugungslinien:

1. Visuell aufgelöst:

| Röntgenbeugungslinie | 2$\ominus$ [°] | $I_A^{rel}$ [%] |
|---|---|---|
| $A^1$ | 8,4 | 5 |
| $A^2$ | 14,8 | 2 |
| $A^3$ | 22,4 | 100 |
| $A^4$ | 23,6 | 22 |
| $A^5$ | 27,2 | 41 |
| $A^6$ | - | - |
| $A^7$ | 35,2 | 14 |
| $A^8$ | - | - |
| $A^9$ | 45,5 | 19 |
| $A^{10}$ | 48,9 | 25 |

2. Mathematisch aufgelöst:

| Röntgenbeugungslinie | 2$\ominus$ [°] | $I_F^{rel}$ [%] | FWHM [°] |
|---|---|---|---|
| $A^{1*}$ | 8,3 | 11 | 3,4 |

(fortgesetzt)

| Röntgenbeugungslinie | 2$\ominus$ [°] | $I_F^{rel}$ [%] | FWHM [°] |
|---|---|---|---|
| A$^{2*}$ | 14,5 | 3 | 3,5 |
| A$^{3*}$ | 22,4 | 28 | 0,5 |
| A$^{4*}$ | 22,9 | 16 | 2,2 |
| A$^{5*}$ | 27,1 | 100 | 4,4 |
| A$^{6*}$ | 31,5 | 36 | 5,7 |
| A$^{7*}$ | 35,3 | 40 | 4,8 |
| A$^{8*}$ | - | - | - |
| A$^{9*}$ | 45,5 | 9 | 0,9 |
| A$^{10*}$ | 48,8 | 33 | 2,3 |

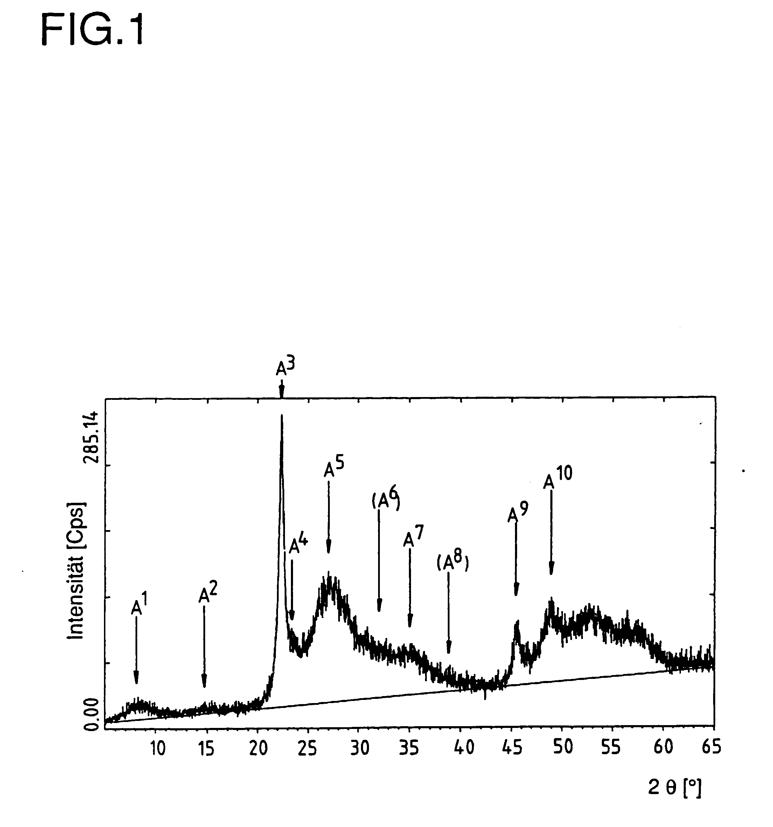

Das die atomare Raumanordnung des Multimetalloxides MI1 reflektierende experimentell ermittelte Cu-K$\alpha$-Pulver-Röntgenbeugungsspektrum zeigt die Figur 1 (Ordinate: Intensität, angegeben als absolute Zählrate; Abszisse: 2$\ominus$-Bereich von 5° bis 65°).

Ferner zeigt die Figur 1 den im Rahmen der Auswertung zu substrahierenden linearen Untergrund (die Verbindungslinie zwischen A(2$\ominus$ = 5°) und A(2$\ominus$ = 65°)), sowie die Lage der einzelnen Beugungslinien A$^i$. Die Berührungspunkte zwischen Grundlinie und Röntgenbeugungsspektrum teilen letzteres in natürlicher Weise in die zwei 2$\ominus$-Bereiche 5° bis 43° und 43° bis 65°.

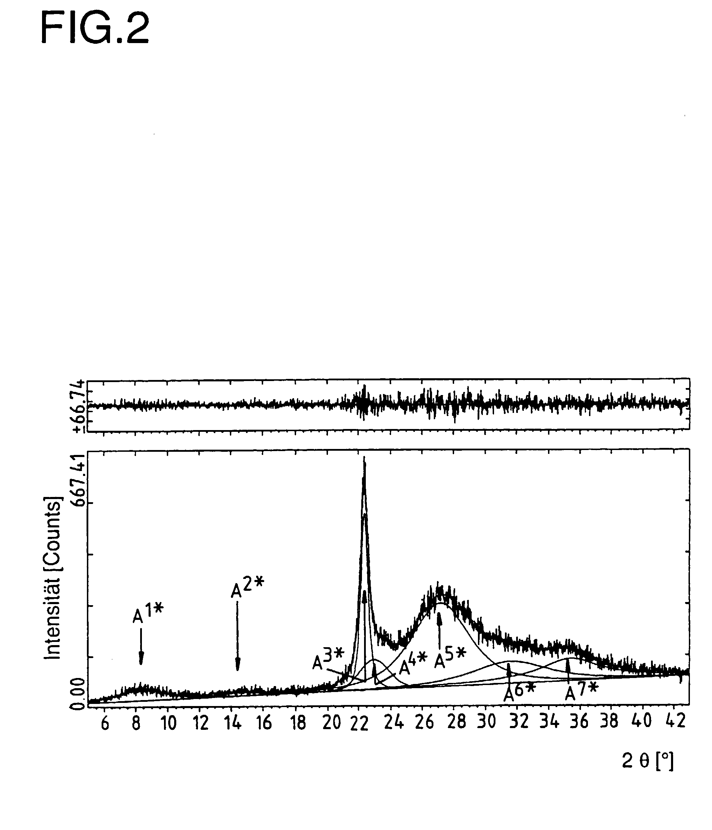

Die Figur 2 zeigt eine Vergrößerung des 2$\ominus$-Bereichs 5° bis 43° des Cu-K$\alpha$-Pulver-Röntgenbeugungsspektrums des Multimetalloxids MI1. Des weiteren zeigt die Figur 2 das Ergebnis (einschließlich der Untergrundlinie) der für diesen Bereich beschreibungsgemäß durchgeführten mathematischen Anpassung und Auflösung (PROFILE, Pearson-VII-Profilfunktion, fixierter Untergrund). Die Superposition der mathematisch erzeugten Beugungslinien A$i^*$ ergibt die Anpassung an die experimentelle Umrißlinie.

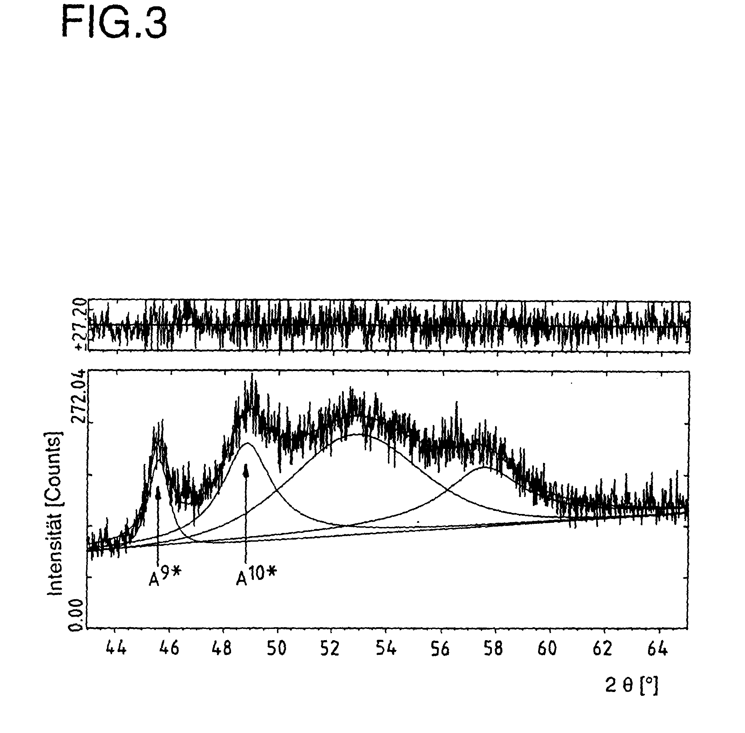

Figur 3 zeigt in entsprechender Weise den vergrößerten 2$\ominus$-Bereich von 43° bis 65° und die für diesen Bereich durchgeführte Entfaltung, einschließlich der linearen Untergrundlinie.

Der oberhalb des eigentlichen Röntgenbeugungsspektrums zu sehende Rechteckausschnitt in den Figuren 2, 3 zeigt jeweils die Differenzlinie zwischen experimenteller Umrißlinie des Pulver-Röntgenbeugungsspektrums und ihrer mathematischen Anpassung. Diese Differenz ist ein Maß für die Güte der Anpassung.

$$\text{MI2:} \qquad Mo_{8,54}V_{2,47}W_{0,99}O_{33,53}$$

Wie für MI1 wurde ein Granulat erzeugt. 400 g des Granulats wurden in einem nach dem Prinzip eines Treibstrahlreaktors arbeitenden Calcinationsofen calciniert. Dabei befand sich das eingesetzte Granulat in einer Schütthöhe von 5 cm auf einem Drahtnetz, das von unten mit einem Gasgemisch durchströmt wurde. Das Volumen des Ofens betrug 3 l, das Kreislaufverhältnis [(im Kreis geführter Gasgemisch-Volumenstrom) : (frisch zugeführter Gasgemisch-Volumenstrom)] wurde zu 20 gewählt. Das eingesetzte Granulat wurde in dem genannten Ofen zunächst innerhalb von 1 h kontinuierlich von 25° auf 325°C erhitzt. Anschließend wurde das eingesetzte Granulat während 4 h bei 325°C gehalten. Zu Beginn der Calcination war das durchströmende Gasgemisch zusammengesetzt aus 120 Nl/h $N_2$, 10 Nl/h Luft und 3,3 Nl/h $NH_3$. Abschließend wurde das eingesetzte Granulat innerhalb von 20 min von 325°C auf 400°C erhitzt und dann noch 1 h bei 400°C gehalten. In dieser Abschlußphase wurde mit einem Gasgemisch aus 120 Nl/h $N_2$ und 10 Nl/h Luft durchströmt. Durch Abschaltung der Wärmezufuhr wurde auf Raumtemperatur abgekühlt.

Es wurde ein pulverförmiges Multimetalloxid MI2 erhalten, das hinsichtlich der $V^{5+,4+,3+}$-Analyse sowie hinsichtlich seines Cu-K$\alpha$-Pulver-Röntgenbeugungsspektrums mit dem Multimetalloxid MI1 identisch war.

$$\text{MI3:} \qquad Mo_{8,35}V_{2,60}W_{1,05}O_{33,40}$$

852,78 g Ammoniumheptamolybdathydrat (MoO$_3$-Gehalt: 81,0 Gew.-%, Idealzusammensetzung: $(NH_4)_6Mo_7O_{24}$ x 4 $H_2O$), 177,14 g Ammoniummetavanadat ($V_2O_5$-Gehalt: 77,0 Gew.-%, Idealzusammensetzung: $NH_4VO_3$) und 156,29 g Ammoniumparawolframathydrat (WO$_3$-Gehalt: 89,0 Gew.-%, Idealzusammensetzung:

$(NH_4)_{10}W_{12}O_{41}$ x 7 $H_2O$) wurden in der genannten Reihenfolge nacheinander bei einer Temperatur von 95°C in 5 l Wasser gelöst. Die erhaltene gelb- bis orangefarbene Lösung wurde auf 80°C abgekühlt und bei einer Eingangstemperatur von 300°C und einer Ausgangstemperatur von 110°C sprühgetrocknet. 100 g des sprühgetrockneten Pulvers wurden anschließend in einem waagrechten Drehkugelofen mit einem isotherm beheizten Quarzkugelvolumen von 1 1 und einer Drehgeschwindigkeit von 12 Umdrehungen pro Minute unter den nachfolgend definierten Bedingungen calciniert. In einem ersten Schritt wurde das eingesetzte Pulver innerhalb von 50 Minuten kontinuierlich von 25°C auf 275°C aufgeheizt, in einem sich an den ersten Schritt unmittelbar anschließenden zweiten Schritt wurde das eingesetzte Pulver innerhalb von 30 Minuten kontinuierlich von 275°C auf 325°C aufgeheizt, in einem sich an den zweiten Schritt unmittelbar anschließenden dritten Schritt wurde das eingesetzte Pulver 4 Stunden lang bei 325°C gehalten und in einem sich an den dritten Schritt unmittelbar anschließenden vierten Schritt wurde das eingesetzte Pulver innerhalb von 3,5 Stunden von 325°C auf 400°C aufgeheizt. Während der ersten drei Schritte wurde die Quarzkugel von einem Gasgemisch bestehend aus 9,6 Nl/h Luft, 3 Nl/h $NH_3$ und 87,4 Nl/h $N_2$ (Gesamtgasfluß: 100 Nl/h) durchströmt. Während des vierten Schrittes wurde die Quarzkugel von einem Gasgemisch bestehend aus 9,6 Nl/h Luft und 87,4 Nl/h $N_2$ (Gesamtgasfluß = 97 Nl/h) durchströmt. Nach Beendigung des vierten Schrittes wurde die äußere Heizung des Drehkugelofens abgeschaltet und durch Anblasen mit Luft von außen gekühlt. Dabei kühlte das eingesetzte Pulver unter dem die Quarzkugel unverändert durchströmenden Gasgemisch aus 9,6 Nl/h Luft und 87,4 Nl/h $N_2$ innerhalb von 5 Stunden auf 25°C ab.

Das resultierende pulverförmige Multimetalloxid MI3 wies eine schwarze Farbe und eine spezifische Oberfläche (DIN 66131) von 0,5 $m^2$/g auf. Das im Multimetalloxid MI3 enthaltene Vanadium lag zu mehr als 99 % als $V^{4+}$ vor. Damit wies das Multimetalloxid MI3 die Stöchiometrie $Mo_{8,35}V_{2,60}W_{1,05}O_{33,40}$ auf.

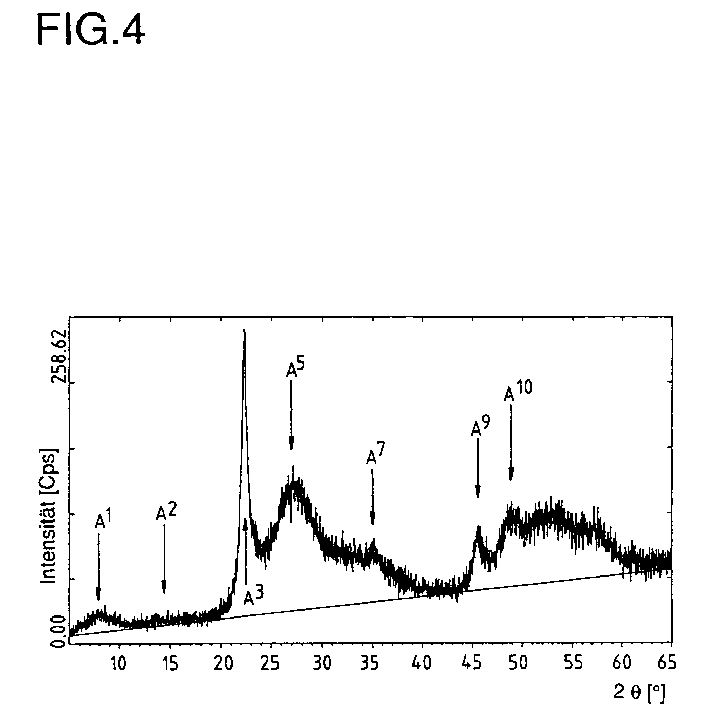

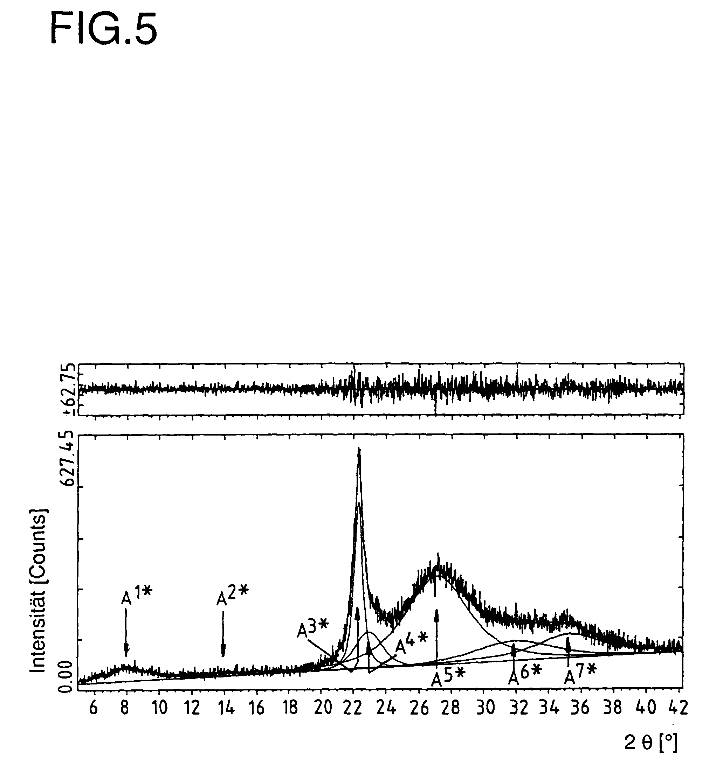

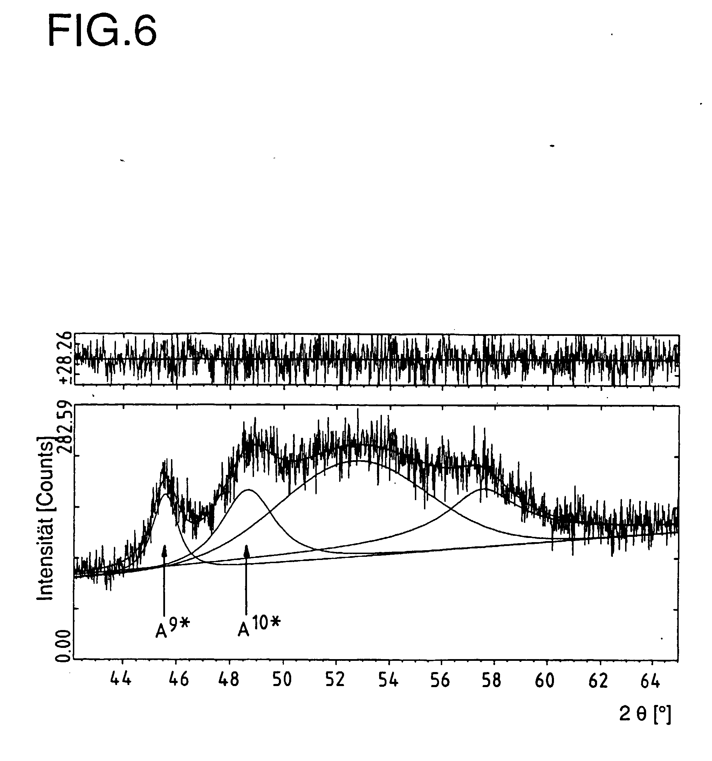

Das zugehörige Cu-K$\alpha$-Pulver-Röntgenbeugungsspektrum zeigen die Figuren 4 bis 6. Es weist die mit MI1 gemeinsame atomare Raumanordnung aus. Seine wie für MI1 durchgeführte Auswertung ergab im relevaten 2$\ominus$-Bereich die nachfolgenden Beugungslinien.

1. Visuell aufgelöst:

| Röntgenbeugungslinie | 2$\ominus$ [°] | $I_A^{rel}$ [%] |
|---|---|---|
| $A^1$ | 7,9 | 7 |
| $A^2$ | 14,7 | kaum zu erkennen |
| $A^3$ | 22,3 | 100 |
| $A^4$ | - | - |
| $A^5$ | 27,1 | 45 |
| $A^6$ | - | - |
| $A^7$ | 35,0 | 17 |
| $A^8$ | - | - |
| $A^9$ | 45,5 | 20 |
| $A^{10}$ | 49,0 | 25 |

2. Mathematisch aufgelöst:

| Röntgenbeugungslinie | 2$\ominus$ [°] | $I_F^{rel}$ [%] | FWHM [°] |
|---|---|---|---|
| $A^{1*}$ | 8,0 | 12 | 3,4 |
| $A^{2*}$ | 14,0 | 2 | 3,1 |
| $A^{3*}$ | 22,3 | 28 | 0,6 |
| $A^{4*}$ | 22,9 | 16 | 2,0 |
| $A^{5*}$ | 27,1 | 100 | 4,6 |

(fortgesetzt)

| Röntgenbeugungslinie | $2\ominus$ [°] | $I_F^{rel}$ [%] | FWHM [°] |
|---|---|---|---|
| A[6*] | 31,9 | 25 | 5,7 |
| A[7*] | 35,2 | 34 | 5,0 |
| A[8*] | - | - | - |
| A[9*] | 45,5 | 10 | 1,1 |
| A[10*] | 48,6 | 20 | 2,4 |

MI4:  $Mo_{8,35}V_{2,60}W_{1,05}O_{33,40}$

Wie für MI3 wurde ein sprühgetrocknetes Pulver erzeugt. 60 g des sprühgetrockneten Pulvers wurden anschließend in einem waagrechten Drehkugelofen mit einem isotherm beheizten Quarzkugelvolumen von 1 l und einer Drehgeschwindigkeit von 12 Umdrehungen pro Minute unter den nachfolgend definierten Bedingungen calciniert. In einem ersten Schritt wurde das eingesetzte Pulver innerhalb von 50 Minuten von 25°C auf 275°C aufgeheizt, in einem sich an den ersten Schritt unmittelbar anschließenden zweiten Schritt wurde das eingesetzte Pulver innerhalb von 30 Minuten kontinuierlich von 275°C auf 325°C aufgeheizt, in einem sich an den zweiten Schritt unmittelbar anschließenden dritten Schritt wurde das eingesetzte Pulver 3 h lang bei 325°C gehalten und in einem sich an den dritten Schritt unmittelbar anschließenden vierten Schritt wurde das eingesetzte Pulver innerhalb von 90 Minuten von 325°C auf 400°C aufgeheizt sowie anschließend nach 10 Minuten bei 400°C gehalten. Während der ersten 3 Schritte wurde die Quarzkugel von einem Gasgemisch bestehend aus 4,8 Nl/h Luft, 1,5 Nl/h $NH_3$ und 43,7 Nl/h $N_2$ (Gesamtgasfluß: 50 Nl/h) durchströmt. Während des vierten und fünften Schrittes wurde die Quarzkugel von einem Gasgemisch bestehend aus 4,8 Nl/h Luft und 43,7 Nl/h $N_2$ (Gesamtgasfluß: 48,5 Nl/h) durchströmt. Nach Beendigung des fünften Schrittes wurde die äußere Heizung des Drehkugelofens abgeschaltet und durch Anblasen mit Luft von außen gekühlt. Dabei kühlte das eingesetzte Pulver unter dem die Quarzkugel unverändert durchströmenden Gasgemisch aus 4,8 Nl/h Luft und 43,7 Nl/h $N_2$ innerhalb von 5 Stunden auf 25°C ab.

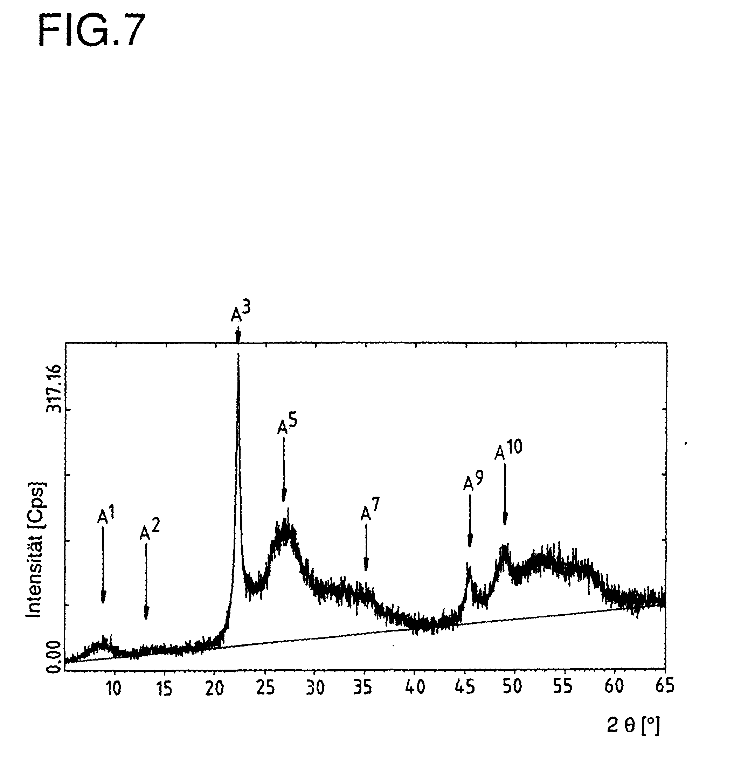

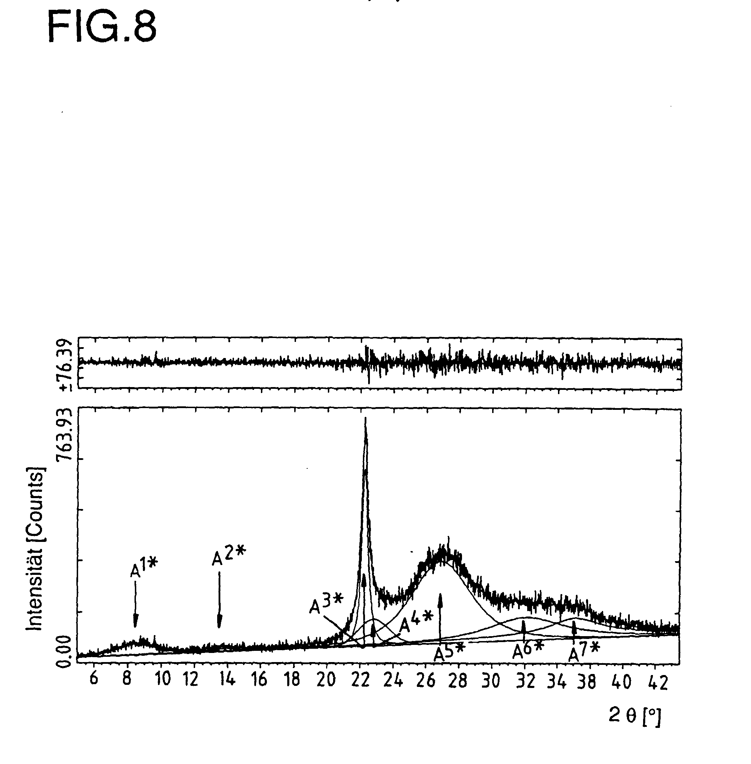

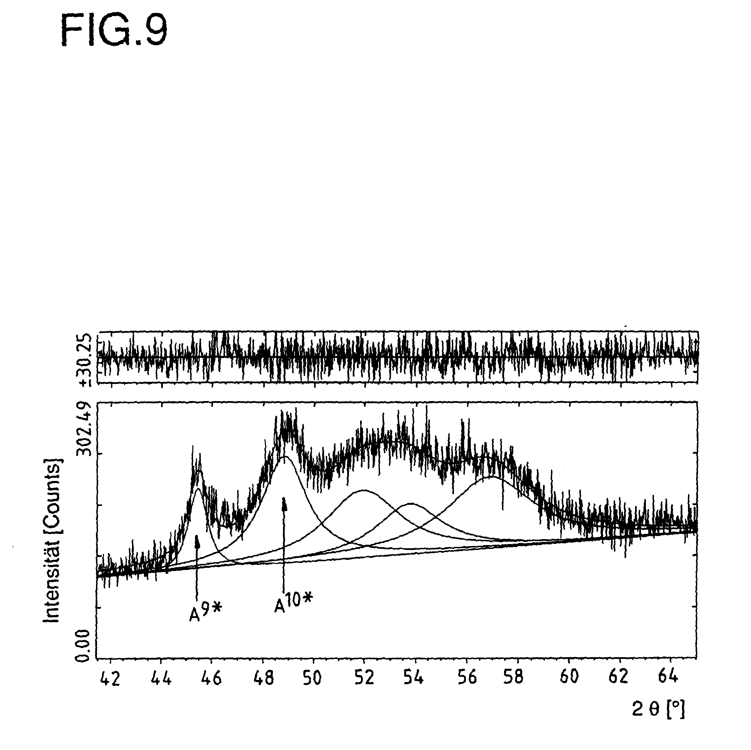

Es wurde ein pulverförmiges Multimetalloxid MI4 erhalten, das hinsichtlich der $V^{5+}, V^{4+}, V^{3+}$-Analyse sowie hinsichtlich seines Cu-K$\alpha$-Pulver-Röntgenbeugungsspektrums dem Multimetalloxid MI3 gleich war. Die wie beschrieben durchgeführte quantitative Auswertung des Röntgenbeugungsspektrums (dargestellt in den Figuren 7 bis 9) ergab im relevanten $2\ominus$-Bereich:

1. Visuell aufgelöst:

| Röntgenbeugungslinie | $2\ominus$ [°] | $I_A^{rel}$ [%] |
|---|---|---|
| A[1] | 8,9 | 5 |
| A[2] | 13,8 | gerade noch zu erkennen |
| A[3] | 22,3 | 100 |
| A[4] | - | - |
| A[5] | 27,0 | 40 |
| A[6] | - | - |
| A[7] | 35,3 | gerade noch zu erkennen |
| A[8] | - | - |
| A[9] | 45,3 | 18 |
| A[10] | 49,0 | 24 |

2. Mathematisch aufgelöst:

| Röntgenbeugungslinie | $2\ominus$ [°] | $I_F^{rel}$ [%] | FWHM [°] |
|---|---|---|---|
| $A^{1*}$ | 8,5 | 8 | 2,8 |
| $A^{2*}$ | 13,7 | 2 | 2,1 |
| $A^{3*}$ | 22,2 | 26 | 0,4 |
| $A^{4*}$ | 22,8 | 15 | 2,2 |
| $A^{5*}$ | 26,9 | 100 | 4,3 |
| $A^{6*}$ | 32,0 | 42 | 5,6 |
| $A^{7*}$ | 35,1 | 61 | 4,7 |
| $A^{8*}$ | - | - | - |
| $A^{9*}$ | 45,4 | 9 | 0,9 |
| $A^{10*}$ | 48,8 | 34 | 2,2 |

MI5:     $Mo_{8,54}V_{2,47}W_{0,99}O_{33,01}$

800 g des zur Herstellung von MI1 erzeugten Sprühpulvers wurden in einem Kneter (Typ LUK 2,5 der Fa. Werner und Pfleiderer, 7000 Stuttgart, DE) mit einem Nutzvolumen von 2,5 l unter Zusatz von 80 g Essigsäure (100 %ig) und 200 g Wasser 1 h geknetet. Dabei wurden während der ersten 10 Minuten Knetung die 80 g Essigsäure und 80 g Wasser zugesetzt. Die restlichen 120 g Wasser wurden während der übrigen 50 Minuten Knetung zugesetzt. Das resultierende feuchte und schollenförmige Knetprodukt wurde 15 h bei einer Temperatur von 110°C getrocknet und danach durch ein Sieb mit einer Maschenweite von 5 mm gedrückt. 100 g des dabei resultierenden Granulats wurde anschließend in einem waagrechten Drehkugelofen wie bei MI1 beschrieben calciniert.

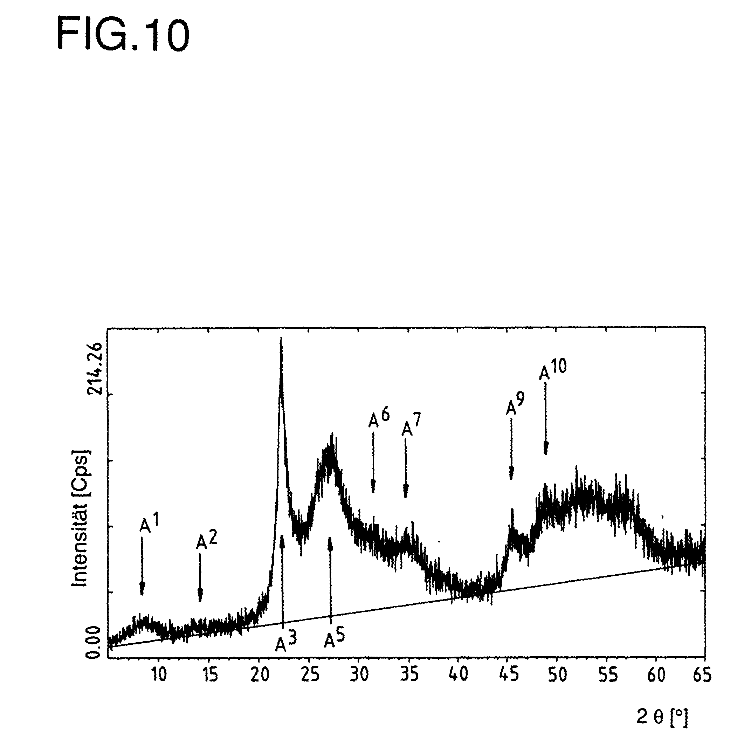

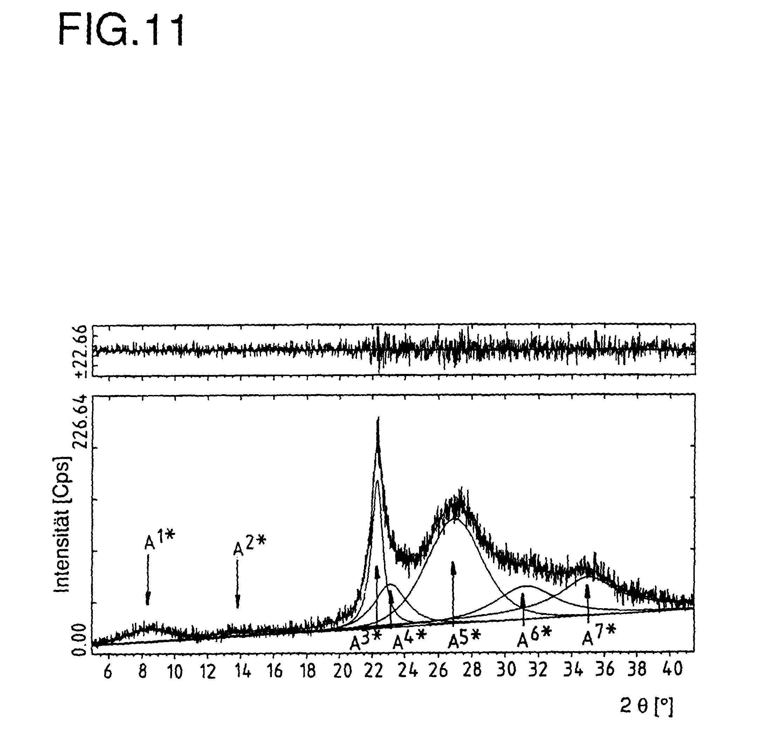

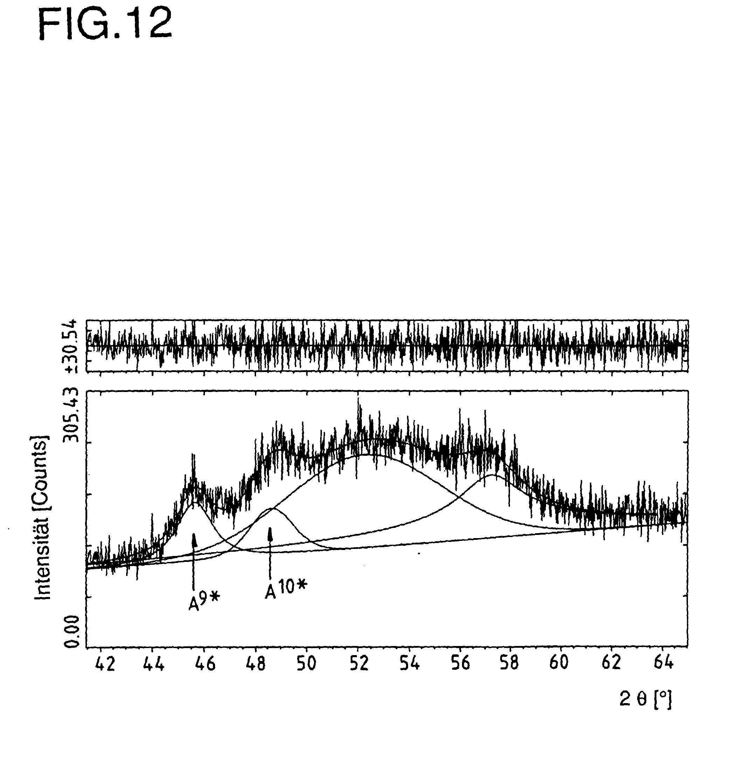

Das resultierende pulverförmige Multimetalloxid MI5 wies eine schwarze Farbe und eine spezifische Oberfläche (DIN 66131) von 16,0 m²/g auf. Das im Multimetalloxid MI5 enthaltene Vanadium lag zu 42 % als $V^{3+}$ und zu 58 % als $V^{4+}$ vor. Damit wies das Multimetalloxid MI5 im Unterschied zum Multimetalloxid MI1 die Stöchiometrie $Mo_{8,54}V_{2,47}W_{0,99}O_{33,01}$ auf. Das wie für das Multimetalloxid MI1 ermittelte und ausgewertete Cu-K$\alpha$-Pulver-Röntgenbeugungsspektrum reflektierte für das Multimetalloxid MI5 dennoch die gleiche atomare Raumanordnung wie für das Multimetalloxid MI1 (vgl. Figuren 10 bis 12). Die ermittelten Beugungslinien im relevanten $2\ominus$-Bereich sind:

1. Visuell aufgelöst:

| Röntgenbeugungslinie | $2\ominus$ [°] | $I_A^{rel}$ [%] |
|---|---|---|
| $A^1$ | 8,6 | 8 |
| $A^2$ | 14,3 | 3 |
| $A^3$ | 22,3 | 100 |
| $A^4$ | - | - |
| $A^5$ | 27,2 | 59 |
| $A^6$ | 31,6 | 26 |
| $A^7$ | 34,8 | 24 |
| $A^8$ | - | - |
| $A^9$ | 45,5 | 24 |
| $A^{10}$ | 49,0 | 31 |

2. Mathematisch aufgelöst:

| Röntgenbeugungslinie | $2\ominus$ [°] | $I_F^{rel}$ [%] | FWHM [°] |
|---|---|---|---|
| $A^{1*}$ | 8,4 | 10 | 3,1 |
| $A^{2*}$ | 13,9 | 2 | 2,0 |
| $A^{3*}$ | 22,3 | 38 | 0,8 |
| $A^{4*}$ | 23,1 | 27 | 2,4 |
| $A^{5*}$ | 26,9 | 100 | 4,0 |
| $A^{6*}$ | 31,2 | 42 | 4,4 |
| $A^{7*}$ | 35,0 | 85 | 4,6 |
| $A^{8*}$ | - | - | - |
| $A^{9*}$ | 45,6 | 15 | 1,6 |
| $A^{10*}$ | 48,6 | 12 | 2,0 |

VMI1: $Mo_{8,54}V_{2,47}W_{0,99}O_{34,18}$

100 g des zur Herstellung von MI5 durch Knetung von Sprühpulver mit Wasser und Essigsäure erhaltenen Granulats wurden wie in Beispiel MI1 beschrieben in einem waagrechten Drehkugelofen calciniert. Anstelle der verschiedenen Gasgemische durchströmte jedoch jeweils eine entsprechende Menge reiner Luft den Drehkugelofen.

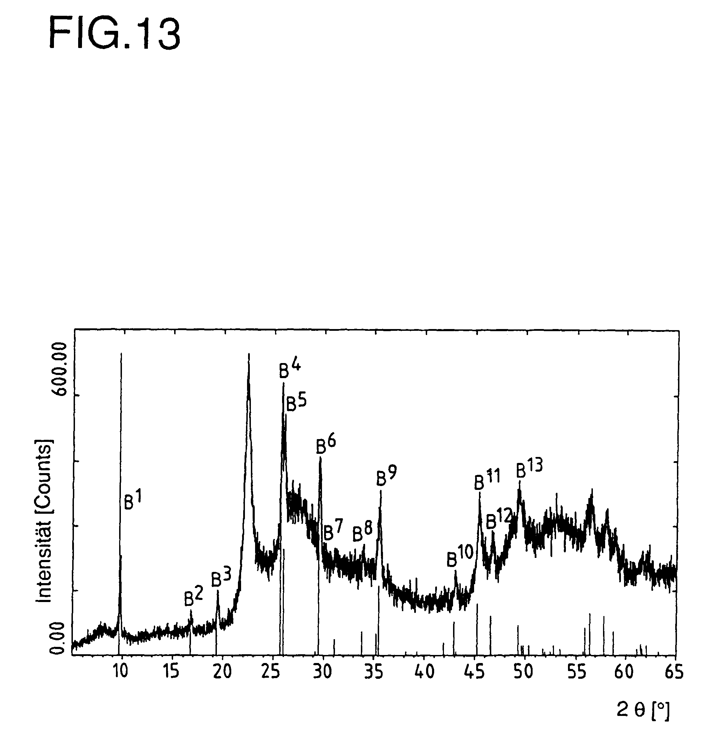

Das resultierende pulverförmige Multimetalloxid VMI1 wies eine schwarze Farbe und eine spezifische Oberfläche (DIN 66131) von 16,2 m$^2$/g auf. Das im Multimetalloxid VMI1 enthaltene Vanadium lag zu 47 % als $V^{4+}$ und zu 53 % als $V^{5+}$ vor. Damit wies das Multimetalloxid VMI1 im Unterschied zum Multimetalloxid MI1 und MI5 die Stöchiometrie $Mo_{8,54}V_{2,47}W_{0,99}O_{34,18}$ auf. Das zugehörige Cu-K$\alpha$-Pulver-Röntgenbeugungsspektrum zeigt die Figur 13. Es ist in offensichtlicher Weise von demjenigen des Multimetalloxids MI1 verschieden und enthält im $2\ominus$-Bereich von 5° bis 50° z.B. nachfolgende (eine relativ geringe Halbwertsbreite aufweisende) intensive Beugungslinien.

Visuell aufgelöst:

| Röntgenbeugungslinie | $2\ominus$ [°] | $I_A^{rel}$ [%] | FWHM [°] |
|---|---|---|---|
| $B^1$ | 9,7 | 32 | 0,15 |
| $B^2$ | 16,8 | 7 | 0,18 |
| $B^3$ | 19,4 | 15 | 0,23 |
| $B^4$ | 25,8 | 91 | 0,19 |
| $B^5$ | 26,1 | 78 | 0,23 |
| $B^6$ | 29,5 | 62 | 0,21 |
| $B^7$ | 31,2 | 24 | 0,45 |
| $B^8$ | 34,0 | 26 | 0,17 |
| $B^9$ | 35,7 | 44 | 0,36 |
| $B^{10}$ | 43,1 | 12 | 0,25 |
| $B^{11}$ | 45,4 | 39 | 0,63 |
| $B^{12}$ | 46,7 | 26 | 0,45 |
| $B^{13}$ | 49,4 | 41 | 1,18 |

D.h. die atomare Raumanordnung des Multimetalloxids VMI1 entspricht nicht derjenigen des Multimetalloxids MI1 oder MI5.

$$MI6: \quad Mo_{9,6}V_{2,4}O_{34,37}$$

Bei 80°C wurden in einem ersten Gefäß 239,74 g Oxalsäuredihydrat (Idealzusammensetzung: $H_2C_2O_4$ x 2 $H_2O$, $H_2C_2O_4$-Gehalt: 71,4 Gew.-%) in 4 l Wasser gelöst. Anschließend wurden unter Aufrechterhaltung der 80°C in der resultierenden wäßrigen Lösung 90,22 g Ammoniumpolyvanadat ($V_2O_5$-Gehalt: 88,2 Gew.-%, Idealzusammensetzung: $(NH_4)_2V_6O_{16}$) unter Rühren aufgelöst, wobei eine tiefblaue wäßrige Lösung A entstand. Das Ammoniumpolyvanadat wurde dabei innerhalb von 15 Minuten in kleinen Portionen zugegeben, um ein Überschäumen des Reaktionsansatzes zu vermeiden. In einem zweiten Gefäß wurden 619,60 g Ammoniumheptamolybdathydrat ($MoO_3$-Gehalt: 81,3 Gew.-%, Idealzusammensetzung: $(NH_4)_6Mo_7O_{24}$ x 4 $H_2O$) in 4 l 80°C heißem Wasser unter Rühren aufgelöst (Lösung B). Anschließend wurde bei 80°C die Lösung B innerhalb von 15 min kontinuierlich in die Lösung A eingerührt. Die dabei resultierende tiefblaue wäßrige Lösung wurde noch 15 h bei 80°C nachgerührt und anschließend sprühgetrocknet (Eingangstemperatur: 310°C, Ausgangstemperatur: 110°C).

100 g des sprühgetrockneten Pulvers wurden in einem waagerechten Drehkugelofen mit einem isotherm beheizten Quarzkugelvolumen von 1 l und einer Drehgeschwindigkeit von 12 Umdrehungen pro Minute unter den nachfolgenden Bedingungen calciniert. In einem ersten Schritt wurde das eingesetzte Pulver innerhalb von 50 Minuten kontinuierlich von 25°C auf 275°C aufgeheizt, in einem sich an den ersten Schritt unmittelbar anschließenden zweiten Schritt wurde das eingesetzte Pulver innerhalb von 35 Minuten kontinuierlich von 275°C auf 325°C aufgeheizt und in einem sich daran unmittelbar anschließenden dritten Schritt während 4 h bei 325°C gehalten. Unmittelbar danach wurde das eingesetzte Pulver in einem vierten Schritt innerhalb von 35 Minuten von 325°C auf 400°C aufgeheizt und in einem sich unmittelbar anschließenden fünften Schritt während 10 Minuten bei 400°C gehalten. Anschließend wurde die äußere Heizung des Drehkugelofens abgeschaltet und die Quarzkugel durch äußeres Anblasen mit Luft abgekühlt. Dabei kühlte sich das eingesetzte Pulver innerhalb von 5 h auf Raumtemperatur (25°C) ab. Während der gesamten Calcinationsdauer (einschließlich der Abkühlphase) wurde die Quarzkugel von einem 50 Nl/h betragenden Luftstrom durchströmt.

Das resultierende pulverförmige Multimetalloxid MI6 wies eine schwarze Farbe und eine spezifische Oberfläche (DIN 66131) von 6,6 $m^2/g$ auf. Das im Multimetalloxid MI6 enthaltene Vanadium lag zu 36 % als $V^{4+}$ und zu 64 % als $V^{5+}$ vor. Damit wies das Multimetalloxid MI6 die Stöchiometrie $Mo_{9,6}V_{2,4}O_{34,37}$ auf.

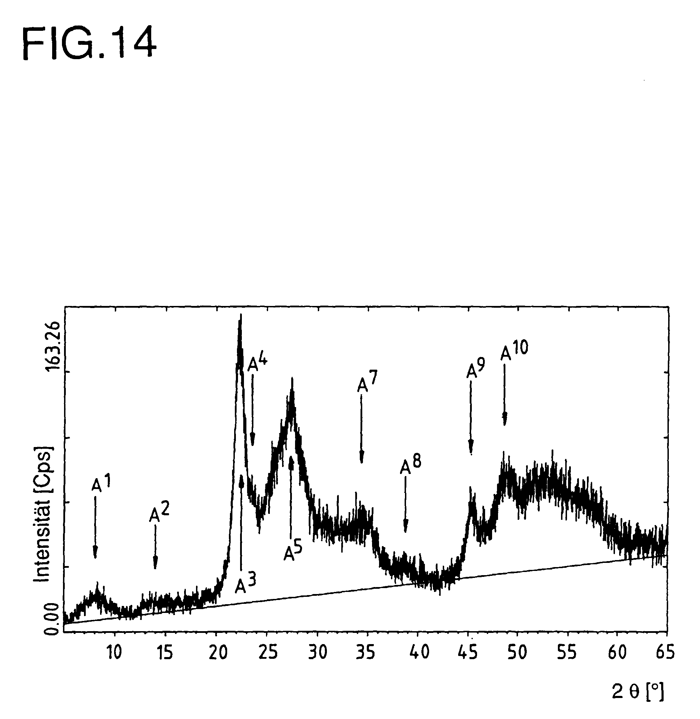

Das visuelle Erscheinungsbild des zugehörigen Cu-K$\alpha$-Pulver-Röntgenbeugungsspektrums entsprach jenem von MI1 (vgl. Figur 14) und wies die mit MI1 gemeinsame atomare Raumanordnung aus. Seine wie für MI1 durchgeführte Auswertung ergab die nachfolgenden Beugungslinien im relevanten 2$\ominus$-Bereich:

1. Visuell aufgelöst:

| Röntgenbeugungslinie | 2$\ominus$ [°] | $I_A^{rel}$ [%] |
|---|---|---|
| $A^1$ | 8,0 | 9 |
| $A^2$ | 14,1 | 4 |
| $A^3$ | 22,3 | 100 |
| $A^4$ | 23,5 | 40 |
| $A^5$ | 27,4 | 74 |
| $A^6$ | - | - |
| $A^7$ | 34,5 | 26 |
| $A^8$ | 38,7 | 12 |
| $A^9$ | 45,4 | 27 |
| $A^{10}$ | 48,9 | 38 |

2. Mathematisch aufgelöst:

| Röntgenbeugungslinie | $2\ominus$ [°] | $I_F^{rel}$ [%] | FWHM [°] |
|---|---|---|---|
| $A^{1*}$ | 8,0 | 5 | 2,7 |
| $A^{2*}$ | 13,8 | 2 | 2,3 |
| $A^{3*}$ | 22,2 | 19 | 0,9 |
| $A^{4*}$ | 23,0 | 23 | 2,6 |
| $A^{5*}$ | 27,3 | 100 | 3,0 |
| $A^{6*}$ | 32,8 | 51 | 7,8 |
| $A^{7*}$ | 34,8 | 4 | 1,8 |
| $A^{8*}$ | - | - | - |
| $A^{9*}$ | 45,3 | 8 | 1,3 |
| $A^{10*}$ | 48,6 | 16 | 2,3 |

MI7:     $Mo_{9,6}V_{2,4}O_{34,13}$

100 g des für MI6 hergestellten sprühgetrockneten Pulvers wurden in einem waagrechten Drehkugelofen mit einem isotherm beheizten Quarzkugelvolumen von 1 l und einer Drehgeschwindigkeit von 12 Umdrehungen pro Minute unter den nachfolgenden Bedingungen calciniert. In einem ersten Schritt wurde das eingesetzte Pulver innerhalb von 50 min von 25°C auf 275°C aufgeheizt, in einem sich an den ersten Schritt unmittelbar anschließenden zweiten Schritt wurde das eingesetzte Pulver innerhalb von 25 Minuten kontinuierlich von 275°C auf 300°C aufgeheizt und in einem sich unmittelbar anschließenden dritten Schritt wurde das eingesetzte Pulver noch während 4 h bei 300°C gehalten. Anschließend wurde die äußere Heizung des Drehkugelofens abgeschaltet und die Drehkugel durch Anblasen mittels Luft abgekühlt. Dabei kühlte sich das eingesetzte Pulver innerhalb von 3 h auf Raumtemperatur (25°C) ab. Während der gesamten Calcinationsdauer (einschließlich der Abkühlphase) wurde die Quarzkugel von einem 50 Nl/h betragenden Luftstrom durchströmt.

Das resultierende pulverförmige Multimetalloxid MI7 wies eine schwarze Farbe und eine spezifische Oberfläche (DIN 66131) von 5,7 m$^2$/g auf. Das im Multimetalloxid MI6 enthaltene Vanadium lag zu 56 % als $V^{4+}$ und zu 44 % als $V^{5+}$ vor. Damit wies das Multimetalloxid MI7 die Stöchiometrie $Mo_{9,6}V_{2,4}O_{34,13}$ auf.

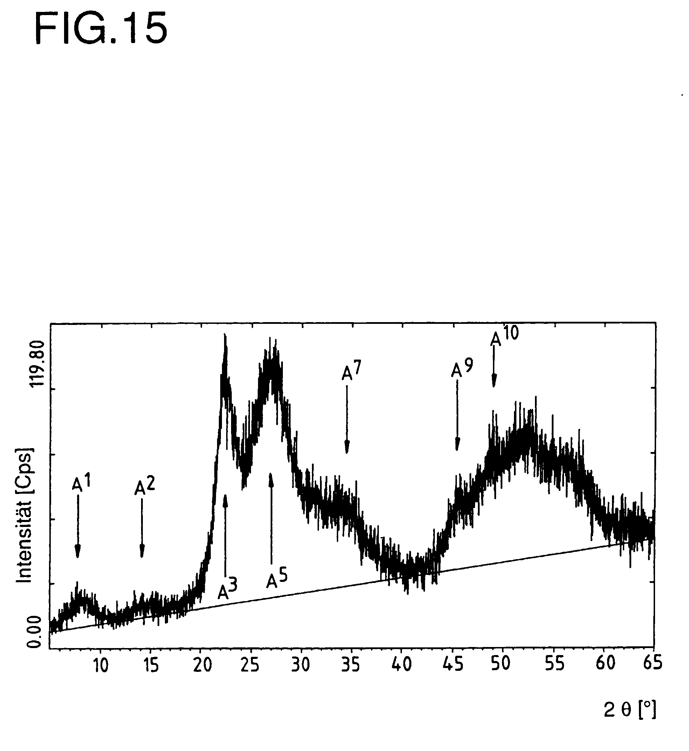

Das visuelle Erscheinungsbild des zugehörigen Cu-K$\alpha$-Pulver-Röntgenbeugungsspektrums entsprach jenem von MI1 (vgl. Figur 15) und wies damit die mit MI1 gemeinsame atomare Raumanordnung aus.

Seine wie für MI1 durchgeführte Auswertung ergab die nachfolgenden Beugungslinien im relevanten $2\ominus$-Bereich:

1. Visuell aufgelöst:

| Röntgenbeugungslinie | $2\ominus$ [°] | $I_A^{rel}$ [%] |
|---|---|---|
| $A^1$ | 7,9 | 10 |
| $A^2$ | 14,2 | 5 |
| $A^3$ | 22,3 | 100 |
| $A^4$ | - | - |
| $A^5$ | 27,1 | 94 |
| $A^6$ | - | - |
| $A^7$ | 34,7 | 30 |

(fortgesetzt)

| Röntgenbeugungslinie | $2\ominus$ [°] | $I_A{}^{rel}$ [%] |
|---|---|---|
| $A^8$ | - | - |
| $A^9$ | 45,6 | 29 |
| $A^{10}$ | 49,0 | 48 |

2. Mathematisch aufgelöst:

| Röntgenbeugungslinie | $2\ominus$ [°] | $I_F{}^{rel}$ [%] | FWHM [°] |
|---|---|---|---|
| $A^{1*}$ | 7,9 | 7 | 3,1 |
| $A^{2*}$ | 14,3 | 4 | 3,0 |
| $A^{3*}$ | 22,3 | 39 | 2,0 |
| $A^{4*}$ | - | - | - |
| $A^{5*}$ | 26,8 | 100 | 4,8 |
| $A^{6*}$ | 31,3 | 25 | 2,9 |
| $A^{7*}$ | 34,2 | 53 | 5,5 |
| $A^{8*}$ | - | - | - |
| $A^{9*}$ | 45,4 | 8 | 2,2 |
| $A^{10*}$ | 48,6 | 29 | 3,9 |

MI8:     $Mo_{9,0}V_{3,0}O_{33,72}$

Bei 80°C wurden in einem ersten Gefäß 306,86 g Oxalsäuredihydrat (Idealzusammensetzung: $H_2C_2O_4$ x 2 $H_2O$, $H_2C_2O_4$-Gehalt: 71,4 Gew.-%) in 4 1 Wasser gelöst. Anschließend wurden unter Aufrechterhaltung der 80°C in der klaren wäßrigen Lösung 115,48 g Ammoniumpolyvanadat ($V_2O_5$-Gehalt: 88,2 Gew.-%, Idealzusammensetzung: $(NH_4)_2V_6O_{16}$) unter Rühren aufgelöst, wobei eine tiefblaue wäßrige Lösung entstand (Lösung A). Das Ammoniumpolyvanadat wurde dabei innerhalb von 15 min in kleinen Portionen zugegeben, um ein Überschäumen des Reaktionsansatzes zu vermeiden. In einem zweiten Gefäß wurden 594,82 g Ammoniumheptamolybdathydrat ($MoO_3$-Gehalt: 81,3 Gew.-%, Idealzusammensetzung: $(NH_4)_6Mo_7O_{24}$ x 4 $H_2O$) in 4 l 80°C heißem Wasser unter Rühren aufgelöst (Lösung B). Anschließend wurde bei 80°C die Lösung B innerhalb von 15 min kontinuierlich in die Lösung A eingerührt. Die dabei resultierende tiefblaue wäßrige Lösung wurde noch 15 h bei 80°C nachgerührt und anschließend sprühgetrocknet (Eingangstemperatur: 310°C, Ausgangstemperatur: 110°C).

100 g des sprühgetrockneten Pulvers wurden in einem waagrechten Drehkugelofen mit einem isotherm beheizten Quarzkugelvolumen von 1 l und einer Drehgeschwindigkeit von 12 Umdrehungen pro Minute wie bei MI7 beschrieben im Luftstrom calciniert.

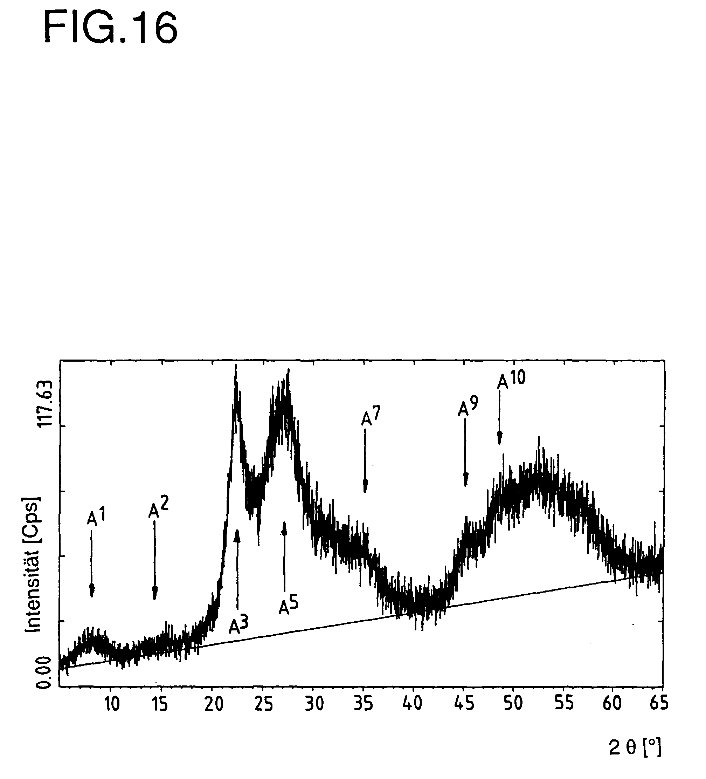

Das resultierende pulverförmige Multimetalloxid MI8 wies eine schwarze Farbe und eine spezifische Oberfläche (DIN 66131) von 5,3 m2/g auf. Das im Multimetalloxid MI8 enthaltene Vanadium lag zu 52 % als $V^{4+}$ und zu 48 % als $V^{5+}$ vor. Damit wies das Multimetalloxid MI8 die Stöchiometrie $Mo_{9,0}V_{3,0}O_{33,72}$ auf. Das visuelle Erscheinungsbild des zugehörigen Cu-K$\alpha$-Pulver-Röntgenbeugungsspektrums entsprach jenem von MI1 (vgl. Figur 16) und wies demgemäß eine mit MI1 gemeinsame atomare Raumanordnung aus. Seine wie für MI1 durchgeführte Auswertung ergab die nachfolgenden Beugungslinien im relevanten $2\ominus$-Bereich:

1. Visuell aufgelöst:

| Röntgenbeugungslinie | $2\ominus$ [°] | $I_A^{rel}$ [%] |
|---|---|---|
| $A^1$ | 8,3 | 9 |
| $A^2$ | 14,6 | 3 |
| $A^3$ | 22,3 | 100 |
| $A^4$ | - | - |
| $A^5$ | 27,0 | 95 |
| $A^6$ | - | - |
| $A^7$ | 35,0 | 29 |
| $A^8$ | - | - |
| $A^9$ | 45,2 | 31 |
| $A^{10}$ | 48,6 | 41 |

2. Mathematisch aufgelöst:

| Röntgenbeugungslinie | $2\ominus$ [°] | $I_F^{rel}$ [%] | FWHM [°] |
|---|---|---|---|
| $A^{1*}$ | 8,0 | 5 | 3,1 |
| $A^{2*}$ | - | - | - |
| $A^{3*}$ | 22,3 | 24 | 1,8 |
| $A^{4*}$ | 23,9 | 11 | 1,1 |
| $A^{5*}$ | 26,9 | 100 | 5,1 |
| $A^{6*}$ | 31,9 | 11 | 3,4 |
| $A^{7*}$ | 34,9 | 26 | 4,1 |
| $A^{8*}$ | - | - | - |
| $A^{9*}$ | 45,2 | 8 | 2,3 |
| $A^{10*}$ | 48,3 | 13 | 3,0 |

MI9:     $Mo_{9,0}V_{3,0}O_{34,04}$

100 g des für MI8 hergestellten sprühgetrockneten Pulvers wurden in einem waagrechten Drehkugelofen mit einem isotherm beheizten Quarzkugelvolumen von 1 l und einer Drehgeschwindigkeit von 12 Umdrehungen pro Minute wie bei MI6 beschrieben im Luftstrom calciniert. Das resultierende Multimetalloxid MI9 wies eine schwarze Farbe und eine spezifische Oberfläche (DIN 66131) von 5,7 m²/g auf.

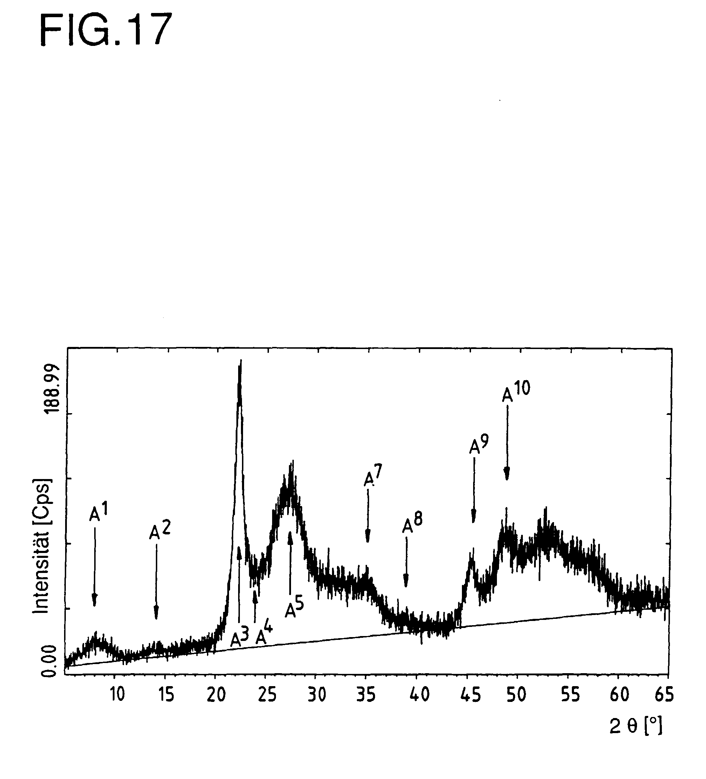

Das im Multimetalloxid MI9 enthaltene Vanadium lag zu 31 % als $V^{4+}$ und zu 69 % als $V^{5+}$ vor. Damit wies das Multimetalloxid MI9 die Stöchiometrie $Mo_{9,0}V_{3,0}O_{34,04}$ auf. Das visuelle Erscheinungsbild des zugehörigen Cu-K$\alpha$-Pulver-Röntgenbeugungsspektrums (Figur 17) entsprach jenem von MI1 und wies die mit MI1 gemeinsame atomare Raumanordnung aus. Seine wie für MI1 durchgeführte Auswertung ergab die nachfolgenden Beugungslinien im relevanten $2\ominus$-Bereich:

1. Visuell aufgelöst:

| Röntgenbeugungslinie | $2\ominus$ [°] | $I_A^{rel}$ [%] |
|---|---|---|
| $A^1$ | 8,0 | 8 |
| $A^2$ | 14,2 | 2 |
| $A^3$ | 22,3 | 100 |
| $A^4$ | 23,9 | 29 |
| $A^5$ | 27,4 | 59 |
| $A^6$ | - | - |
| $A^7$ | 35,0 | 20 |
| $A^8$ | 38,8 | 9 |
| $A^9$ | 45,4 | 23 |
| $A^{10}$ | 48,8 | 32 |

2. Mathematisch aufgelöst:

| Röntgenbeugungslinie | $2\ominus$ [°] | $I_F^{rel}$ [%] | FWHM [°] |
|---|---|---|---|
| $A^{1*}$ | 8,1 | 9 | 3,1 |
| $A^{2*}$ | 14,0 | 9 | 2,2 |
| $A^{3*}$ | 22,2 | 43 | 0,9 |
| $A^{4*}$ | 23,3 | 37 | 2,6 |
| $A^{5*}$ | 27,2 | 100 | 3,9 |
| $A^{6*}$ | 31,8 | 25 | 3,9 |
| $A^{7*}$ | 35,1 | 90 | 4,1 |
| $A^{8*}$ | - | - | - |
| $A^{9*}$ | 45,3 | 10 | 1,4 |
| $A^{10*}$ | 48,6 | 24 | 2,5 |

VMI2:     $Mo_{9,0}V_{3,0}O_{32,90}$

100 g des sprühgetrockneten Pulvers aus MI8 wurden in einem waagrechten Drehkugelofen mit einem isotherm beheizten Quarzkugelvolumen von 1 l und einer Drehgeschwindigkeit von 12 Umdrehungen pro Minute wie bei MI1 beschrieben calciniert.

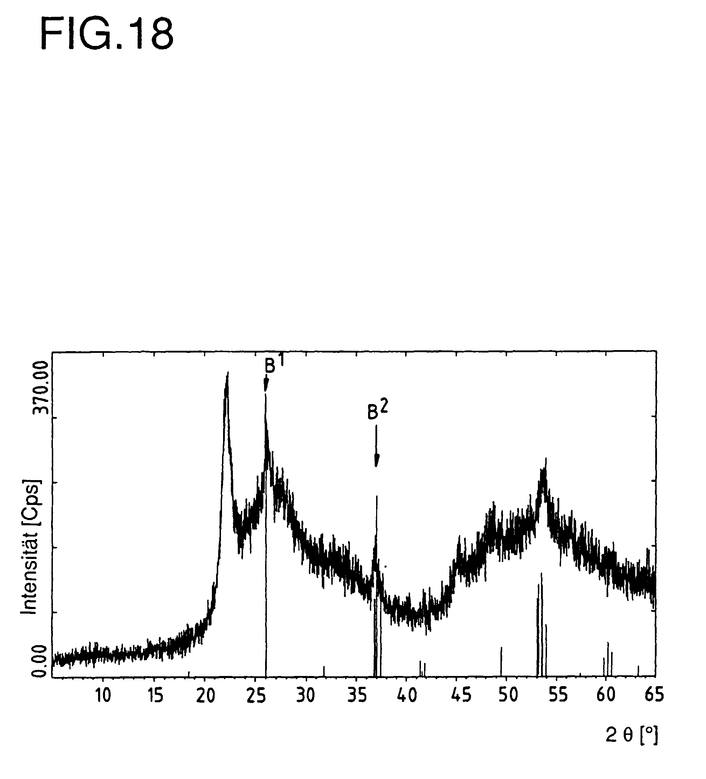

Das resultierende Multimetalloxid VMI2 wies eine schwarze Farbe und eine spezifische Oberfläche (DIN 66131) von 6,8 $m^2$/g auf. Das im Multimetalloxid VMI2 enthaltene Vanadium lag zu 93 % als $V^{4+}$ und zu 7 % als $V^{3+}$ vor. Das visuelle Erscheinungsbild des zugehörigen Cu-K$\alpha$-Pulver-Röntgenbeugungsspektrums war in offensichtlicher Weise von demjenigen des Multimetalloxids MI1 verschieden (vgl. Figur 18) und enthielt im $2\ominus$-Bereich von 5° bis 50° z.B. nachfolgende intensive Beugungslinien:

Visuell ausgewertet:

| Röntgenbeugungslinie | 2$\Theta$ [°] | $I_A^{rel}$ [%] | FWHM [°] |
|---|---|---|---|
| B1 | 26,0 | 85 | 0,50 |
| B2 | 36,9 | 31 | 0,45 |

2) Herstellung von Multimetalloxiden II

MII1: Ausgangsmasse 1 ($Cu_{12}Mo_{12}O_{48}$) von M5 der DE-A 4 440 891 wurde nachgearbeitet:

In 500 ml Wasser wurden 55,3 g Cu(II)-Oxid (CuO, Fa. Merck, Darmstadt, reinst, mindestens 96 %, pulverförmig) und 100,0 g Mo(VI)-Oxid ($MoO_3$, Fa. Merck, Darmstadt, p.a., mindestens 99,5 %, pulverförmig) eindispergiert. Die Gesamtmenge der wäßrigen Dispersion wurde in einem Autoklaven (Werkstoff: Hastelloy C4; Innenvolumen: 2,5 l) unter Rühren (1000 Umdrehungen pro Minute) auf 350°C erwärmt und bei dieser Temperatur und dem zugehörigen Überdruck während 24 h unter Rühren gehalten. Anschließend wurde der Autoklav auf Raumtemperatur abgekühlt, die darin enthaltene wäßrige Dispersion entnommen, der dispergierte Feststoff abfiltriert und anschließend im Trokkenschrank bei 80°C getrocknet. Das resultierende trockene Pulver wies bei der rasterelektronenmikroskopischen Untersuchung (REM) kristalline Partikel mit einem zahlenmittleren Korngrößendurchmesser von etwa 8 μm aus. Die chemische Analyse der kristallinen Partikel ergab ein Cu/Mo-Verhältnis von ca. 1.

Unter Anwendung von Cu-K$\alpha$-Strahlung (Siemens-Diffraktometer D-5000, 40 kV, 30 mA, mit automatischer Divergenz-, Streustrahl- und Zählrohrblende und Peltier-Detektor) zeigte das kristalline Pulver CuMoOy das nachfolgende Röntgenbeugungsmuster, wiedergegeben in Gestalt von von der Wellenlänge der verwendeten Röntgenstrahlung unabhängigen Netzebenenabständen d[Å], sowie den zugehörigen, auf die intensitätsstärkste (Amplitude) Beugungslinie bezogenen, relativen Intensitäten (%) der verschiedenen Beugungslinien:

| d [Å] | $I_A^{rel}$ [%] |
|---|---|
| 2,44 | 100 |
| 3,01 | 58,4 |
| 3,14 | 56,8 |
| 2,75 | 35,5 |
| 2,82 | 30,6 |
| 3,39 | 30,1 |
| 1,65 | 25,2 |
| 3,96 | 21,6 |
| 1,72 | 21,1 |
| 2,50 | 20,5 |
| 2,20 | 17,3 |
| 4,68 | 15,2 |
| 2,48 | 14,5 |
| 1,96 | 13,8 |
| 3,71 | 13,7 |
| 3,75 | 13,2 |
| 1,80 | 12,4 |
| 2,90 | 12,2 |
| 2,34 | 12,1 |
| 1,61 | 11,8 |

(fortgesetzt)

| d [Å] | $I_A^{rel}$ [%] |
|---|---|
| 1,59 | 11,6 |
| 3,31 | 11,5 |
| 1,85 | 11,5 |
| 2,04 | 11,3 |
| 2,08 | 11,2 |
| 1,70 | 11,1 |
| 2,00 | 10,8 |
| 1,89 | 10,7 |
| 2,12 | 10,3 |
| 1,88 | 9,15 |
| 1,86 | 8,52 |
| 1,98 | 8,25 |
| 2,30 | 8,01 |
| 2,04 | 7,29 |
| 2,66 | 6,89 |
| 1,57 | 6,73 |
| 1,55 | 6,54 |
| 1,77 | 6,53 |
| 2,37 | 6,45 |
| 1,56 | 6,03 |
| 1,55 | 5,93 |
| 3,45 | 5,82 |
| 2,12 | 5,79 |
| 1,63 | 5,76 |
| 2,06 | 5,72 |
| 1,83 | 5,43 |
| 1,60 | 5,42 |
| 2,14 | 5,12 |
| 5,81 | 4,91 |

Die Ungenauigkeit der Angabe der Netzebenenabstände d beläuft sich auf ± 0,20 Å (die intensitätsarmen Linien umfassen vermutlich auch auf geringfügige Verunreinigungen zurückgehende Linien). Dieses Röntgenbeugungsmuster entspricht demjenigen für Cu-MoO$_4$-III in Russian Journal of Inorganic Chemistry 36 (7), 1991, S. 927, Table 1.

MII2:  Ausgangsmasse 1 (Cu$_{12}$Mo$_6$W$_6$O$_{42-48}$) von M3 der DE-A 19528646 wurde nachgearbeitet:

223,05 g Ammoniumheptamolybdathydrat (MoO$_3$-Gehalt: 81,3 Gew.-%, Idealzusammensetzung: (NH$_4$)$_6$Mo$_7$O$_{24}$x4H$_2$O) und 327,52 g Ammoniumparawolframathydrat (WO$_3$-Gehalt: 89,2 Gew.-%, Idealzusammensetzung: (NH$_4$)$_{10}$W$_{12}$O$_{41}$x7H$_2$O) wurden bei 90°C in 5 l Wasser unter Rühren gelöst (Lösung A). 492,64 g Kupferacetathydrat (Cu-Gehalt: 32,5 Gew.-%, Idealzusammensetzung: Cu(CH$_3$COO)$_2$xH$_2$O) wurden mit 3 l Wasser und 197,88 g einer 25 gew.-%igen wäßrigen Ammoniaklösung versetzt und 15 min bei 25°C gerührt, wobei eine hellblaue Suspension erhalten wurde (Suspension B). Anschließend wurde die Suspension B in die 90°C aufweisende Lösung A eingerührt und die dabei resultierende Suspension 3 h bei 80°C nachgerührt. Das wäßrige Suspendiermedium der resultierenden wäßrigen Suspension (Suspension C) wies nach Abkühlen auf 25°C einen pH-Wert (Glaselektrode) von 5,3 auf. Die Suspension C wurde bei einer Eingangstemperatur von 310°C und einer Austrittstemperatur von 110°C sprühgetrocknet.

Das dabei erhaltene grüne Pulver wurde an Luft calciniert, wobei in einem ersten Schritt innerhalb von 24 h kontinuierlich von 25°C auf 300°C und in einem sich daran anschließenden zweiten Schritt innerhalb von 3 h kontinuierlich von 300 auf 780°C erhitzt, sowie in einem dritten Schritt die Temperatur noch 1 h bei 780°C gehalten wurde. Die Calcination wurde in einem Drehkugelofen mit einem nutzbaren Volumen von 1 l durchgeführt, wobei jeweils 60 g Ausgangssprühpulver eingesetzt und ein Luftstrom von 50 Nl/h eingestellt wurde.

Das resultierende Pulver wies eine braune Farbe und eine spezifische Oberfläche nach DIN 66131 von 0,3 $m^2/g$ sowie die Zusammensetzung $Cu_{12}Mo_6W_6O_{42-48}$ auf. Bei der REM-Untersuchung wies das Pulver kristalline Partikel mit einem zahlenmittleren Korngrößendurchmesser von etwa 8 μm aus. Unter Anwendung von Cu-Kα-Strahlung (Siemens-Diffraktometer D-5000, 40 kV, 30 mA, mit automatischer Divergenz-Streustrahl- und Zählrohrblende sowie Peltier-Detektor) zeigte das kristalline Pulver ein Pulver-Röntgendiagramm, das eine Superposition des Wolframit-Fingerabdrucks mit dem HT-Cu-Molybdat-Fingerabdruck zeigte, d.h. es besaß einen zweiphasigen Aufbau. Gemäß den Linienintensitäten lagen die beiden Strukturtypen etwa im Häufigkeitsverhältnis 60 (Wolframit-Struktur): 40 (HT-Cu-Molybdat-Typ) vor.

Der HT-Cu-Molybdat-Strukturtyp ist durch nachfolgendes Röntgenbeugungsdiagramm, aufgenommen an einem kristallinen Pulver der Zusammensetzung $CuMo_{0,9}W_{0,1}O_{3,5-4}$ unter Anwendung von Cu-Kα-Strahlung (Siemens-Diffraktometer D 5000, 48 kV, 30 mA, mit automatischer Divergenz-, Streustrahl- und Zählrohrblende sowie Peltier-Detektor) und wiedergegeben in Gestalt von von der Wellenlänge der verwendeten Röntgenstrahlung unabhängigen Netzebenenabständen d[Å], sowie den zugehörigen, auf die intensitätsstärkste (Amplitude) Beugungslinie bezogenen, relativen Intensitäten (%) der verschiedenen Beugungslinien (angeordnet nach abnehmender Intensität) charakterisiert:

| d [Å] | $I_A^{rel}$ [%] |
|---|---|
| 3,40 | 100 |
| 3,54 | 72 |
| 2,27 | 39 |
| 6,79 | 32 |
| 2,56 | 25 |
| 1,57 | 22 |
| 1,87 | 19 |
| 2,96 | 18 |
| 3,56 | 18 |
| 1,64 | 17 |
| 2,66 | 16 |
| 1,59 | 16 |
| 1,55 | 16 |
| 2,67 | 14 |
| 2,00 | 14 |
| 3,04 | 13 |
| 1,57 | 11 |
| 2,36 | 11 |
| 1,44 | 11 |
| 1,70 | 10 |
| 1,51 | 10 |

(fortgesetzt)

| d [Å] | $I_A^{rel}$ [%] |
|---|---|
| 2,35 | 10 |
| 1,55 | 9,6 |
| 2,32 | 9,2 |
| 2,89 | 9,0 |
| 1,68 | 9,0 |
| 1,48 | 8,9 |
| 1,94 | 8,7 |
| 1,60 | 8,7 |
| 2,69 | 8,6 |
| 1,84 | 8,5 |
| 1,99 | 8,1 |
| 3,92 | 8,0 |
| 2,34 | 8,0 |
| 2,70 | 7,5 |
| 1,52 | 7,5 |
| 1,49 | 7,4 |
| 2,44 | 7,3 |
| 5,78 | 7,2 |
| 1,68 | 7,1 |
| 1,91 | 6,9 |
| 1,71 | 6,8 |
| 1,74 | 6,5 |
| 4,56 | 6,3 |
| 3,16 | 6,1 |
| 2,08 | 5,7 |
| 2,02 | 5,6 |
| 2,28 | 5,6 |
| 2,05 | 5,5 |
| 1,80 | 5,4 |
| 5,13 | 4,9 |
| 3,48 | 4,9 |
| 3,12 | 4,1 |
| 4,20 | 3,7 |
| 4,39 | 3,5 |
| 3,84 | 3,5 |
| 3,73 | 3,4 |
| 4,68 | 3,4 |
| 4,46 | 3,2 |

(fortgesetzt)

| d [Å] | $I_A^{rel}$ [%] |
|---|---|
| 3,76 | 2,9 |

Die Ungenauigkeit der Angabe der Netznebenenabstände d beläuft sich für d-Werte $\geq$ 2,9 Å im wesentlichen auf $\pm$ 0,3 Å und für d-Werte < 2,9 Å im wesentlichen auf $\pm$ 0,2 Å (die intensitätsarmen Linien umfassen eventuell auch auf geringfügige Verunreinigungen zurückgehende Linien).

3) Herstellung von Schalenkatalysatoren S1 bis S3 und Vergleichsschalenkatalysatoren SV1 bis SV3

S1: Nach Mahlen des Multimetalloxids MI1 aus 1) ($Mo_{8,54}V_{2,47}W_{0,99}O_{33,53}$) auf Teilchendurchmesser im Bereich von 0,1 bis 50 µm wurden mit dem dabei resultierenden Aktivmassenpulver in einer Drehtrommel unporöse, oberflächenrauhe Steatitkugeln eines Durchmessers von 4 bis 5 mm und einer Oberflächenrauhigkeit $R_Z$ im Bereich von 40 bis 200 µm (bestimmt gemäß DIN 4768, Blatt 1, mit einem Hommel Tester für DIN-/ISO-Oberflächenmeßgrößen der Hommelwerke, DE) in einer Menge von 50 g Pulver je 200 g Steatitkugeln bei gleichzeitigem Zusatz von 18 g Wasser gemäß der DE-A 4442346 beschichtet. Anschließend wurde mit 110°C heißer Luft getrocknet.

SV1: Wie S1, als Aktivmasse wurde jedoch das in entsprechender Weise gemahlene Multimetalloxid VMI1 aus 1), das ebenfalls die Zusammensetzung $Mo_{8,54}V_{2,47}W_{0,99}O_{33,53}$ aufwies, verwendet.

S2: Wie S1, als Aktivmasse wurde jedoch ein Gemisch aus in entsprechender Weise gemahlenem Multimetalloxid MI1 aus 1) ($Mo_{12}V_{3,47}W_{1,39}O_{47,11}$) und dem pulverförmigen auf einen zahlenmittleren Korngrößtdurchmesser von 1 bis 3 µm mit einer Zentrifugalmühle der Fa. Retsch, DE, gemahlenen Multimetalloxid MII1 aus 2) ($Cu_{12}Mo_{12}O_{48}$) verwendet. Dabei wurde von MII1 soviel ins gemahlene MI1 eingerührt, daß das molare Verhältnis der vorgenannten stöchiometrischen Einheiten im resultierenden Mischpulver 6,5 (MI1):1 (MII1) betrug. Eine innige Vermischung der beiden Pulver wurde durch Vermischen in einem Intensivmischer der Fa. Gustav Eirich, Hardheim, DE (Typ RO2, MPM-System) erzielt. Unter Verwendung eines 10 l Volumen aufweisenden, mit 64 Umdrehungen pro Minute rotierenden Tellers und eines Wirbelwerkzeugs "Stern", welches zum Teller gegenläufig mit 900 Umdrehungen pro Minute rotierte, wurden 600 g Gemisch 15 Minuten gemischt.

Die resultierende zweiphasige Aktivmasse war:

$$[Mo_{12}V_{3,47}W_{1,39}O_{47,11}]_{6,5}[Cu_{12}Mo_{12}O_{48}] \, ] \triangleq Mo_{12}V_3W_{1,2}Cu_{1,6}O_{47,23}$$

S3: Wie S2, anstelle des Multimetalloxids MII1 aus 2) wurde jedoch das Multimetalloxid MII2 aus 2) eingesetzt.

Die resultierende Aktivmasse war:

$$[Mo_{12}V_{3,47}W_{1,39}O_{47,11}]_{6,5}[Cu_{12}Mo_6W_6O_{42\text{-}48}].$$

SV2: Wie S1, als Aktivmasse wurde jedoch ein wie folgt erzeugtes feinteiliges Pulver verwendet:

127 g Kupfer(II)acetatmonohydrat (32,4 Gew.-% Cu) wurden in 2700 g Wasser zu einer Lösung I gelöst. In 5500 g Wasser wurden bei 95°C nacheinander 860 g Ammoniumheptamolybdattetrahydrat (81,3 Gew.-% $MoO_3$), 143 g Ammoniummetavanadat (77,2 Gew.-% $V_2O_5$) und 126 g Ammoniumparawolframatheptahydrat (89,3 Gew.-% $WO_3$) zu einer Lösung II gelöst. Anschließend wurde die Lösung I auf einmal in die Lösung II eingerührt und das wäßrige, auf 80°C temperierte Gemisch bei einer Austrittstemperatur von 110°C sprühgetrocknet.

800 g des Sprühpulvers wurden wie die 800 g Sprühpulver zur Herstellung von MI1 geknetet und calciniert. Danach wurde auf einen Teilchendurchmesser im Bereich von 0,1 bis 50 µm gemahlen und wie in S1 ein Schalenkatalysator geformt.

Stöchiometrie der Aktivmasse: $Mo_{12}V_3W_{1,2}Cu_{1,6}O_{x^*}$ .

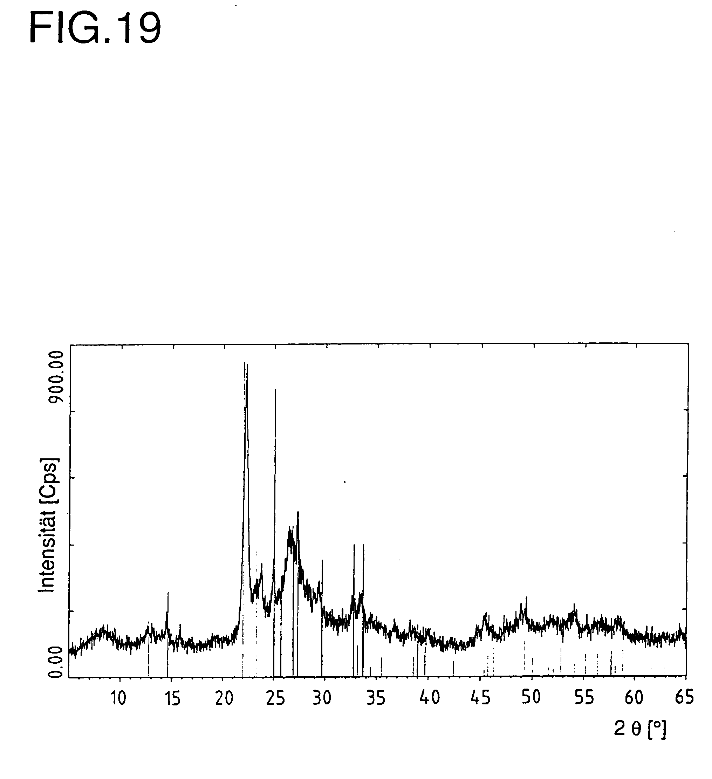

Fig. 19 zeigt das zugehörige Cu-K$\alpha$-Pulver-Röntgenbeugungsspektrum, das von demjenigen des Multimetalloxids MI1 signifikant dadurch verschieden ist, daß es im Bereich 2$\ominus$ = 5° bis 50° von A$^1$ bis A$^{10}$ verschiedene Röntgenbeugungslinien aufweist.

SV3:   Wie SV2, während der gesamten Calcinationsdauer durchströmte jedoch ausschließlich 3,6 Nl/h Luft den Calcinationsofen.

Stöchiometrie der Aktivmasse: $Mo_{12}V_3W_{1,2}Cu_{1,6}O_x$**.

4. Verwendung der Schalenkatalysatoren aus 3) als Katalysatoren für die Gasphasenoxidation von Acrolein zu Acrylsäure

Die Katalysatoren wurden in einen Rohrreaktor gefüllt (V2A-Stahl, 25 mm Innendurchmesser, 2000 g Katalysatorschüttung, Salzbadtemperierung) und bei Reaktionstemperaturen im Bereich von 250 bis 270°C unter Anwendung einer Verweilzeit von 2,0 sec mit einem gasförmigen Gemisch der Zusammensetzung
5 Vol.-% Acrolein,
7 Vol.-% Sauerstoff,
10 Vol.-% Wasserdampf und
78 Vol.-% Stickstoff beschickt. Die Salzbadtemperatur wurde in allen Fällen so eingestellt, daß, nach beendeter Formierung, bei einfachem Durchgang ein einheitlicher Acroleinumsatz von 99 % resultierte. Das aus dem Reaktor strömende Produktgasgemisch wurde gaschromatographisch analysiert. Die Ergebnisse für die Selektivität der Acrylsäurebildung in Anwendung der verschiedenen Katalysatoren zeigt die nachfolgende Tabelle.

| Katalysator | S % | Badtemperatur (°C) |
| --- | --- | --- |
| S1 | 92,4 | 255 |
| SV1 | 92,2 | 264 |
| S2 | 96,0 | 254 |
| S3 | 96,5 | 255 |
| SV3 | 95,1 | 261 |
| SV2 | 95,3 | 254 |

Umsatz, Selektivität und Verweilzeit sind dabei wie folgt definiert:

$$\text{Umsatz U an Acrolein (\%)} = \frac{\text{Molzahl umgesetztes Acrolein}}{\text{Molzahl eingesetztes Acrolein}} \times 100;$$

$$\text{Selektivität S der Acrylsäurebildung \%} = \frac{\text{Molzahl Acrolein umgesetzt zu Acrylsäure}}{\text{Molzahl Acrolein insgesamt umgesetzt}} \times 100;$$

$$\text{Verweilzeit (sec)} = \frac{\text{mit Katalysator gefülltes Leervolumen des Reaktors (1)}}{\text{durchgesetzte Synthesegasmenge (Nl/h)}} \times 3600.$$

## Patentansprüche

1.   Multimetalloxide der allgemeinen Formel I

$$Mo_{12-a-b-c}\, Va\, M^1_b\, M^2_c\, O_x \qquad (I),$$

in der die Variablen folgende Bedeutung haben:

$M^1$ =   W und/oder Nb,
$M^2$ =   Ti, Zr, Zr, Hf, Ta, Cr, Si und/oder Ge,
a =   0,1 bis 6,

b = 0 bis 6,

c = 0 bis 6,

mit der Maßgabe, daß a + b + c = 0,1 bis 6 und

x = eine Zahl, die durch die Wertigkeit und Häufigkeit der von Sauerstoff verschiedenen Elemente in I bestimmt wird,

die dadurch gekennzeichnet sind, daß

ihre atomare Raumanordnung unter Anwendung von Cu-K$\alpha$-Strahlung ($\lambda$ = 1,54178 Å) ein Pulver-Röntgenbeugungsspektrum (die Intensität A der gebeugten Röntgenstrahlung aufgetragen als Funktion des zweifachen Beugungswinkels (2$\ominus$)) bedingt, das im 20-Bereich von 5° bis 50° mindestens die nachfolgenden charakteristischen Beugungslinien $A^1$, $A^3$, $A^5$, $A^9$ und $A^{10}$ jedoch höchstens die nachfolgenden Beugungslinien $A^1$ bis $A^{10}$ enthält:

| Röntgenbeugungslinie | 2$\ominus$ [°] |
|---|---|
| $A^1$ | 8,3 ± 0,7 |
| $A^2$ | 14,4 ± 0,7 |
| $A^3$ | 22,3 ± 0,2 |
| $A^4$ | 23,5 ± 0,7 |
| $A^5$ | 27,2 ± 0,4 |
| $A^6$ | 32,0 ± 0,8 |
| $A^7$ | 34,8 ± 0,6 |
| $A^8$ | 38,7 ± 0,5 |
| $A^9$ | 45,4 ± 0,4 |
| $A^{10}$ | 48,8 ± 0,4 |

2. Multimetalloxide nach Anspruch 1, wobei die Beugungslinien $A^1$ bis $A^{10}$ die nachfolgenden relativen Amplituden ($I_A^{rel}$[%]) aufweisen:

| | $I_A^{rel}$[%] |
|---|---|
| $A^1$ | 7 ± 5 |
| $A^2$ | 5 ± 5 |
| $A^3$ | 100 |
| $A^4$ | 40 ± 40 |
| $A^5$ | 70 ± 40 |
| $A^6$ | 25 ± 25 |
| $A^7$ | 20 ± 20 |
| $A^8$ | 10 ± 10 |
| $A^9$ | 25 ± 15 |
| $A^{10}$ | 35 ± 20 |

3. Multimetalloxide nach Anspruch 1 oder 2, wobei das Cu-K$\alpha$-Pulver-Röntgenbeugungsspektrum keine hochaufgelöste Beugungslinie aufweist, deren Halbwertsbreite (im 20-Maßstab) < 0,25° beträgt.

4. Multimetalloxide nach Anspruch 1 bis 3 der allgemeinen Formel I

$$Mo_{12-a-b-c} \, V_a \, M_b^1 \, M_c^2 \, O_x \tag{I},$$

in der die Variablen folgende Bedeutung haben:

$M^1$ =  W und/oder Nb
$M^2$ =  Ti, Zr, Hf, Ta, Cr, Si und/oder Ge
a =  0,1 bis 6,
b =  0 bis 6,
c =  0 bis 6,

mit der Maßgabe, daß a + b + c = 0,1 bis 6 und

x =  eine Zahl, die durch die Wertigkeit und Häufigkeit der von Sauerstoff verschiedenen Elemente in I bestimmt wird,

die dadurch gekennzeichnet sind, daß

ihre atomare Raumanordnung derjenigen des Multimetalloxids $Mo_{8,54}V_{2,47}W_{0,99}O_{33,35}$ entspricht, das wie folgt erhältlich ist:

33,746 kg Ammoniumheptamolybdathydrat ($MoO_3$-Gehalt: 81,8 Gew.-%, Idealzusammensetzung: $(NH_4)_6Mo_7O_{24}$ x 4 $H_2O$), 6,576 kg Ammoniummetavanadat ($V_2O_5$-Gehalt: 76,5 Gew.-%, Idealzusammensetzung: $NH_4VO_3$), 5,764 kg Ammoniumparawolframathydrat ($WO_3$-Gehalt: 89,0 Gew.-%, Idealzusammensetzung: $(NH_4)_{10}W_{12}O_{41}$ x 7 $H_2O$) und 7,033 kg Ammoniumacetat ($CH_3COONH_4$-Gehalt: 97,0 Gew.-%, Idealzusammensetzung: $CH_3COONH_4$) werden in der genannten Reihenfolge nacheinander bei einer Temperatur von 90°C in 250 l Wasser unter Rühren gelöst. Die resultierende gelb- bis organgefarbene Lösung wird auf 80°C abgekühlt und mit einer Eingangstemperatur von 300°C und einer Ausgangstemperatur von 110°C sprühgetrocknet; 800 g des erhaltenen Sprühpulvers werden in einem Kneter (Typ LUK 2,5 der Fa. Werner und Pfleiderer, 7000 Stuttgart, DE) mit einem Nutzvolumen von 2,5 l unter Zusatz von 250 g Wasser 1 h geknetet. Dabei werden von den 250 g Wasser 180 g innerhalb der ersten 10 Minuten der Knetung und 70 g innerhalb der restlichen 50 Minuten der Knetung zugesetzt. Das resultierende feuchte und schollenförmige Knetprodukt wird 15 h bei einer Temperatur von 110°C getrocknet und danach durch ein Sieb mit einer Maschenweite von 5 mm gedrückt; 100 g des dabei resultierenden Granulates werden anschließend in einem waagrechten Drehkugelofen mit einem isotherm beheizten Quarzkugelvolumen von 1 l und einer Drehgeschwindigkeit von 12 Umdrehungen pro Minute calciniert, wobei sich die Calcinationsbedingungen wie folgt gestalten:

1. Schritt:  innerhalb von 50 min das eingesetzte Granulat kontinuierlich von 25 auf 275°C erhitzen;

2. Schritt:  innerhalb von 30 min das eingesetzte Granulat kontinuierlich von 275°C auf 325°C erhitzen;

3. Schritt:  das eingesetzte Granulat 4 h bei 325°C halten;

4. Schritt:  innerhalb von 30 min das eingesetzte Granulat kontinuierlich von 325°C auf 400°C erhitzen;

5. Schritt:  das eingesetzte Granulat 10 min bei 400°C halten;

anschließend wird die äußere Beheizung des Drehkugelofens abgeschaltet und letztere durch äußeres Anblasen mit Umgebungsluft gekühlt; dabei kühlt sich das eingesetzte Granulat innerhalb von 5 h auf 25°C ab; während der einzelnen Calcinationsschritte wird der Innenraum des Drehkugelofens parallel zur Drehachse von Gasmischungen durchströmt, die nachfolgende Zusammensetzungen aufweisen (Normalbedingungen (N) = 1 atm, 25°C):

1. Schritt,  2. Schritt und 3. Schritt: 3,6 Nl/h Luft, 1,5 Nl/h $NH_3$ und 44,9 Nl/h $N_2$ (Gesamtgasfluß: 50 Nl/h);

4. Schritt,  5. Schritt und Abkühlphase: 3,6 Nl/h Luft und 44,9 Nl/h N2 (Gesamtgasfluß: 48,5 Nl/h).

5.  Multimetalloxide nach Anspruch 1 bis 4, wobei a = 0,5 bis 4,5 beträgt.

6.  Multimetalloxide nach Anspruch 1 bis 5, wobei b = 0,1 bis 6 beträgt.

**7.** Multimetalloxide nach Anspruch 1 bis 6, wobei b = 0,5 bis 4 beträgt.

**8.** Multimetalloxide nach Anspruch 1 bis 7, wobei c = 0,1 bis 6 beträgt.

**9.** Multimetalloxide nach Anspruch 1 bis 8, wobei c = 0,5 bis 4 beträgt.

**10.** Multimetalloxide nach Anspruch 1 bis 9, wobei a + b + c = 0,5 bis 4,5 beträgt.

**11.** Multimetalloxide nach Anspruch 1 bis 10, wobei x = 15 bis 50 beträgt.

**12.** Multimetalloxide nach Anspruch 1 bis 11, wobei x = 25 bis 40 beträgt.

**13.** Multimetalloxide nach Anspruch 1 bis 12, wobei x = 30 bis 40 beträgt.

**14.** Multimetalloxide nach Anspruch 1 bis 7 und 10 bis 13, wobei c = 0 und $M^1$ ausschließlich W ist, und wobei wenigstens 25 % des enthaltenen Vanadiums als $V^{4+}$ vorliegen.

**15.** Verfahren der katalytischen Gasphasenoxidation organischer Verbindungen, dadurch gekennzeichnet, daß als Katalysator ein solcher verwendet wird, dessen Aktivmasse ein Multimetalloxid gemäß Anspruch 1 bis 14 ist.

**16.** Verfahren nach Anspruch 15, dadurch gekennzeichnet, daß es das Verfahren der katalytischen Gasphasenoxidation von Acrolein zu Acrylsäure ist.

**17.** Katalysator, dessen Aktivmasse aus einem Multimetalloxid gemäß Anspruch 1 bis 14 besteht.

**18.** Verwendung von Multimetalloxiden gemäß Anspruch 1 bis 14 als Ausgangsverbindung zur Herstellung von Mo, V und wenigstens eines der Elemente W, Nb, Ti, Zr, Hf, Ta, Cr, Si und/oder Ge enthaltenden Multimetalloxidmassen.

**19.** Verfahren zur Herstellung von Mo, V und wenigstens eines der Elemente W, Nb, Ti, Zr, Hf, Ta, Cr, Si und/oder Ge enthaltenden Multimetalloxidmassen, dadurch gekennzeichnet, daß wenigstens ein Multimetalloxid gemäß Anspruch 1 bis 14 als Ausgangsverbindung verwendet wird.

**20.** Feinteiliges Gemisch, enthaltend ein feinteiliges Multimetalloxid I gemäß Anspruch 1 bis 14 sowie ein feinteiliges Multimetalloxid der allgemeinen Formel II

$$M^3_{12} \, Cu_d \, H_e \, O_y \qquad \text{(II)},$$

mit

$M^3 =$ Mo, W, V, Nb und/oder Ta,

$d =$ 4 bis 30,

$e =$ 0 bis 20 und

$y =$ eine Zahl, die durch die Wertigkeit und Häufigkeit der von Sauerstoff verschiedenen Elemente in II bestimmt wird.

**21.** Wenigstens zweiphasige Multimetalloxidmassen der allgemeinen Formel III

$$[A]_p \, [B]_q \qquad \text{(III)},$$

in der die Variablen folgende Bedeutung haben:

$$A = (Mo_{12-a-b-c} \, V_a \, M^1_b \, M^2_c \, O_x) \, x \, \frac{12}{12\text{-}a\text{-}b\text{-}c} \qquad \text{(Aktivphase)}$$

$$B = M^3_{12} \, Cu_d \, H_e \, O_y \qquad \text{(Promotorphase),}$$

$M^1 =$    W und/oder Nb,

$M^2 =$    Ti, Zr, Hf, Ta, Cr, Si und/oder Ge,

$a =$    0,1 bis 6,

$b =$    0 bis 6,

$c =$    0 bis 6,

mit der Maßgabe, daß a + b + c = 0,1 bis 6,

$x =$    eine Zahl, die durch die Wertigkeit und Häufigkeit der von Sauerstoff verschiedenen Elemente in A be-stimmt wird,

$M^3 =$    Mo, W, V, Nb und/oder Ta,

$d =$    4 bis 30,

$e =$    0 bis 20,

$y =$    eine Zahl, die durch die Wertigkeit und Häufigkeit der von Sauerstoff verschiedenen Elemente in B bestimmt wird,

$p, q =$    von null verschiedene Zahlen, deren Verhältnis p/q 160 : 1 bis 1 : 1 beträgt,

die den Anteil $[A]_p$ in Form dreidimensional ausgedehnter, von ihrer lokalen Umgebung aufgrund ihrer von ihrer lokalen Umgebung verschiedenen chemischen Zusammensetzung abgegrenzter Bereiche A der chemischen Zusammensetzung

$$A \qquad Mo_{12-a-b-c} \, V_a \, M^1_b \, M^2_c \, O_x$$

und den Anteil $[B]_q$ in Form dreidimensional ausgedehnter, von ihrer lokalen Umgebung aufgrund ihrer von ihrer lokalen Umgebung verschiedenen chemischen Zusammensetzung abgegrenzter, Bereiche B der chemischen Zusammensetzung

$$B \qquad M^3_{12} \, Cu_d \, H_e \, O_y$$

enthalten, wobei die Bereiche A, B relativ zueinander wie in einem Gemisch aus feinteiligem A und feinteiligem B verteilt sind und die atomare Raumanordnung der Bereiche A unter Anwendung von Cu-Kα-Strahlung (λ = 1,54178 Å) ein Pulver-Röntgenbeugungsspektrum (die Intensität der gebeugten Röntgenstrahlung aufgetragen als Funktion des zweifachen Beugungswinkels (2Θ)) bedingt, das im 2Θ-Bereich von 5° bis 50° mindestens die nachfolgenden charakteristischen Beugungslinien $A^1$, $A^3$, $A^5$, $A^9$ und $A^{10}$, jedoch höchstens die nachfolgenden Beugungslinien $A^1$ bis $A^{10}$ enthält:

| Röntgenbeugungslinie | 2Θ [°] |
|---|---|
| $A^1$ | 8,3 ± 0,7 |
| $A^2$ | 14,4 ± 0,7 |

**EP 0 774 297 B1**

(fortgesetzt)

| Röntgenbeugungslinie | $2\ominus$ [°] |
|---|---|
| $A^3$ | $22{,}3 \pm 0{,}2$ |
| $A^4$ | $23{,}5 \pm 0{,}7$ |
| $A^5$ | $27{,}2 \pm 0{,}4$ |
| $A^6$ | $32{,}0 \pm 0{,}8$ |
| $A^7$ | $34{,}8 \pm 0{,}6$ |
| $A^8$ | $38{,}7 \pm 0{,}5$ |
| $A^9$ | $45{,}4 \pm 0{,}4$ |
| $A^{10}$ | $48{,}8 \pm 0{,}4$ |

**22.** Wenigstens zweiphasige Multimetalloxidmassen der allgemeinen Formel III

$$[A]_p \, [B]_q \tag{III},$$

in der die Variablen folgende Bedeutung haben:

$$A = (Mo_{12-a-b-c} \, V_a \, M^1_b \, M^2_c \, O_x) \times \frac{12}{12-a-b-c} \quad \text{(Aktivphase)},$$

$$B = M^3_{12} \, Cu_d \, H_e \, O_y \quad \text{(Promotorphase)},$$

$M^1 =$     W und/oder Nb,

$M^2 =$     Ti, Zr, Hf, Ta, Cr, Si und/oder Ge,

$a =$     0,1 bis 6,

$b =$     0 bis 6,

$c =$     0 bis 6,

mit der Maßgabe, daß a + b + c = 0,1 bis 6,

$x =$     eine Zahl, die durch die Wertigkeit und Häufigkeit der von Sauerstoff verschiedenen Elemente in A bestimmt wird,

$M^3 =$     Mo, W, V, Nb und/oder Ta,

$d =$     4 bis 30,

$e =$     0 bis 20,

$y =$     eine Zahl, die durch die Wertigkeit und Häufigkeit der von Sauerstoff verschiedenen Elemente in B bestimmt wird,

$p, q =$     von null verschiedene Zahlen, deren Verhältnis p/q 160 : 1 bis 1 : 1 beträgt,

die den Anteil $[A]_p$ in Form dreidimensional ausgedehnter, von ihrer lokalen Umgebung aufgrund ihrer von ihrer lokalen Umgebung verschiedenen chemischen Zusammensetzung abgegrenzter Bereiche A der chemischen Zusammensetzung

$$A \qquad Mo_{12-a-b-c} \, V_a \, M^1_b \, M^2_c \, O_x$$

und den Anteil $[B]_q$ in Form dreidimensional ausgedehnter, von ihrer lokalen Umgebung aufgrund ihrer von ihrer lokalen Umgebung verschiedenen chemischen Zusammensetzung abgegrenzter, Bereiche B der chemischen Zusammensetzung

$$B \qquad M^3_{12} \, Cu_d \, H_e \, O_y$$

enthalten, wobei die Bereiche A, B relativ zueinander wie in einem Gemisch aus feinteiligem A und feinteiligem B verteilt sind und die atomare Raumanordnung der Bereiche A diejenige der Multimetalloxide gemäß Anspruch 4 ist.

23. Multimetalloxidmassen nach Anspruch 21 oder 22, deren Bereiche B den Strukturtyp wenigstens eines der in der nachfolgenden Tabelle 1 aufgelisteten Kupfermolybdate aufweisen (der Ausdruck in Klammern gibt die Quelle für den zugehörigen Röntgenbeugungsfingerabdruck wieder):

Tabelle 1

| | |
|---|---|
| $Cu_3(MoO_4)_2(OH)_2$ | (Lindgrenit, Karteikarte 36-405 der JCPDS-ICDD Kartei (1991)), |
| $Cu_4MoO_6O_{20}$ | (A. Moini et al., Inorg. Chem. 25(21) (1986) S. 3782 bis 3785), |
| $Cu_4Mo_5O_{17}$ | (Karteikarte 39-181 der JCPDS-ICDD Kartei (1991)), |
| $Cu_6Mo_5O_{18}$ | (Karteikarte 40-865 der JCPDS-ICDD Kartei (1991)), |
| $Cu_6Mo_4O_{15}$ | (Karteikarte 35-17 der JCPDS-ICDD Kartei (1991)), |
| $CuMoO_4$ | (Karteikarte 22-242 der JCPDS-ICDD Kartei (1991)), |
| $CuMoO_4$ | (Russian Journal of Inorganic Chemistry 36(7), 1991, S. 927-928, Table 1, $CuMoO_4$-III mit verzerrter Wolframit-Struktur ($CuWO_4$, Karteikarte 21-307 JCPDS-ICDD Kartei (1994)), |
| $CuMoO_4$ | (Karteikarte 26-546 der JCPDS-ICDD Kartei (1991)), |
| HT-Cu-Molybdat | (DE-A 19528646), |
| $Cu_{4-x}Mo_3O_{12}$ | mit x = 0 bis 0,25 (Karteikarte 24-56 und 26-547 der JCPDS-ICDD Kartei (1991)), |
| $Cu_3Mo_2O_9$ | (Karteikarte 24-55 und 34-637 der JCPDS-ICDD Kartei (1991)) und |
| $Cu_2MoO_5$ | (Karteikarte 22-607 der JCPDS-ICDD Kartei (1991)). |

24. Multimetalloxidmassen nach Anspruch 21 bis 23, deren Bereiche A und B Größtdurchmesser im Bereich > 0 bis 300 µm aufweisen.

25. Multimetalloxidmassen nach Anspruch 21 bis 23, deren Bereiche A und B Größtdurchmesser im Bereich 1 bis 30 µm aufweisen.

26. Verfahren zur Herstellung von Katalysatoren mit Multimetalloxidmassen nach Anspruch 21 bis 23 als Aktivmasse, dadurch gekennzeichnet, daß man wenigstens ein feinteiliges Multimetalloxid I gemäß Anspruch 1 bis 14 und wenigstens ein feinteiliges Multimetalloxid der allgemeinen Formel II

$$M^3_{12} \, Cu_d \, H_e \, O_y \qquad\qquad (II),$$

mit

$M^3$ = Mo, W, V, Nb und/oder Ta,

d = 4 bis 30,

e = 0 bis 20 und

y =     eine Zahl, die durch die Wertigkeit und Häufigkeit der von Sauerstoff verschiedenen Elemente in II bestimmt wird,

jeweils getrennt vorbildet, die getrennt vorgebildeten feinteiligen Multimetalloxide I und II zu einem innigen Trockengemisch vermischt und das innige Trockengemisch formt.

27. Verfahren der katalytischen Gasphasenoxidation organischer Verbindungen, dadurch gekennzeichnet, daß als Katalysator ein solcher verwendet wird, dessen Aktivmasse eine Multimetalloxidmasse gemäß Anspruch 21 bis 25 ist.

28. Verfahren nach Anspruch 27, dadurch gekennzeichnet, daß es das Verfahren der katalytischen Gasphasenoxidation von Acrolein zu Acrylsäure ist.

29. Mehrphasige Multimetalloxidmassen, die Phasen aus Multimetalloxiden I gemäß Anspruch 1 bis 14 in feinteiliger homogener Verteilung mit Größtdurchmessern im Bereich > 0 bis 300 µm aufweisen.

**Claims**

1. A multimetal oxide of the formula I

$$\mathrm{Mo}_{12-a-b-c}\, \mathrm{V}_a\, \mathrm{M}^1_b\, \mathrm{M}^2_c\, \mathrm{O}_x \tag{I},$$

where

$M^1$ is W and/or Nb,
$M^2$ is Ti, Zr, Hf, Ta, Cr, Si and/or Ge,
a is from 0.1 to 6,
b is from 0 to 6,
c is from 0 to 6,

with the proviso that a + b + c is from 0.1 to 6, and
     x is a number which is determined by the valency and frequency of the elements in I other than oxygen,
whose three-dimensional atomic arrangement obtained using CuKα radiation ($\lambda$ = 1.54178 Å) gives an X-ray powder diffraction spectrum (the intensity A of the diffracted X-rays plotted as a function of twice the diffraction angle (2$\ominus$)) which contains, in the 2$\ominus$ range from 5 to 50°, at least the following characteristic diffraction lines $A^1$, $A^3$, $A^5$, $A^9$ and $A^{10}$, but at most the following diffraction lines $A^1$ to $A^{10}$:

| X-ray diffraction line | 2$\ominus$ [°] |
|---|---|
| $A^1$ | 8.3 ± 0.7 |
| $A^2$ | 14.4 ± 0.7 |
| $A^3$ | 22.3 ± 0.2 |
| $A^4$ | 23.5 ± 0.7 |
| $A^5$ | 27.2 ± 0.4 |
| $A^6$ | 32.0 ± 0.8 |
| $A^7$ | 34.8 ± 0.6 |
| $A^8$ | 38.7 ± 0.5 |
| $A^9$ | 45.4 ± 0.4 |
| $A^{10}$ | 48.8 ± 0.4 |

2. A multimetal oxide as claimed in claim 1, wherein the diffraction lines $A^1$ to $A^{10}$ have the following relative amplitudes ($I_A^{rel}$[%]):

| | $I_A^{rel}[\%]$ |
|---|---|
| $A^1$ | $7 \pm 5$ |
| $A^2$ | $5 \pm 5$ |
| $A^3$ | 100 |
| $A^4$ | $40 \pm 40$ |
| $A^5$ | $70 \pm 40$ |
| $A^6$ | $25 \pm 25$ |
| $A^7$ | $20 \pm 20$ |
| $A^8$ | $10 \pm 10$ |
| $A^9$ | $25 \pm 15$ |
| $A^{10}$ | $35 \pm 20$ |

3. A multimetal oxide as claimed in claim 1 or 2, wherein the CuK$\alpha$ X-ray powder diffraction spectrum has no highly dissolved diffraction line whose half-width (on the 2$\ominus$ scale) is < 0.25°.

4. A multimetal oxide as claimed in any of claims 1 to 3 of the formula I

$$Mo_{12-a-b-c} V_a M^1_b M^2_c O_x \qquad (I),$$

where

M$^1$ is W or Nb,

M$^2$ is Ti, Zr, Hf, Ta, Cr, Si or Ge,

a is from 0.1 to 6,

b is from 0 to 6,

c is from 0 to 6,

with the proviso that a + b + c is from 0.1 to 6, and
x is a number which is determined by the valency and frequency of the elements in I other than oxygen,
wherein
its three-dimensional atomic arrangement corresponds to that of the multimetal oxide $Mo_{8.54}V_{2.47}W_{0.99}O_{33.35}$ which is obtainable as follows:
33.746 kg of ammonium heptamolybdate hydrate ($MoO_3$ content: 81.8% by weight, ideal composition: $(NH_4)_6Mo_7O_{24} \cdot H_2O$), 6.576 kg of ammonium metavanadate ($V_2O_5$ content: 76.5% by weight, ideal composition: $NH_4VO_3$), 5.764 kg of ammonium paratungstate hydrate ($WO_3$ content: 89.0% by weight, ideal composition: $(NH_4)_{10}W_{12}O_{41} \cdot 7 H_2O$) and 7.033 kg of ammonium acetate ($CH_3COONH_4$ content: 97.0% by weight, ideal composition: $CH_3COONH_4$) are dissolved in succession in the stated order in 250 l of water at 90°C while stirring; the resulting yellow to orange solution is cooled to 80°C and spray-dried at an inlet temperature of 300°C and an outlet temperature of 110°C;
800 g of the spray powder obtained are kneaded in a kneader (type LUK 2.5 from Werner and Pfleiderer, 7000 Stuttgart, Germany) having an effective volume of 2.5 1, with the addition of 250 g of water, for 1 hour. Of the 250 g of water, 180 g are added within the first 10 minutes of kneading and 70 g within the remaining 50 minutes of kneading; the resulting moist and clod-like kneaded product is dried for 15 hours at 110°C and then forced through a sieve having a mesh size of 5 mm;
100 g of the resulting granules are then calcined in a horizontal rotary kiln with an isothermally heated quartz bulb volume of 1 l and a rotary speed of 12 revolutions per minute, the calcination conditions being as follows:

1st step: the granules used are continuously heated from 25 to 275°C within 50 minutes;

2nd step: the granules used are continuously heated from 275 to 325°C within 30 minutes;

3rd step: the granules used are kept at 325°C for 4 hours;

4th step: the granules used are continuously heated from 325 to 400°C within 30 minutes;

5th step: the granules used are kept at 400°C for 10 minutes;

the external heating of the rotary kiln is then switched off and the latter is cooled by blowing on surrounding air from the outside; the granules used cool to 25°C in the course of 5 hours;

during the individual calcination steps, gas mixtures which have the following compositions (standard temperature and pressure conditions (S.T.P.) 1 atm, 25°C) flow through the interior of the rotary kiln, parallel to the axis of rotation:

1st step, 2nd step and 3rd step: 3.6 l (S.T.P.)/h of air, 1.5 l (S.T.P.)/h of $NH_3$ and 44.9 l (S.T.P.)/h of $N_2$ (total gas flow: 50 l (S.T.P.)/h);

4th step, 5th step and cooling phase: 3.6 1 (S.T.P.)/h of air and 44.9 1 (S.T.P.)/h of N2 (total gas flow: 48.5 1 (S.T.P.)/h).

5. A multimetal oxide as claimed in any of claims 1 to 4, wherein a is from 0.5 to 4.5.

6. A multimetal oxide as claimed in any of claims 1 to 5, wherein b is from 0.1 to 6.

7. A multimetal oxide as claimed in any of claims 1 to 6, wherein b is from 0.5 to 4.

8. A multimetal oxide as claimed in any of claims 1 to 7, wherein c is from 0.1 to 6.

9. A multimetal oxide as claimed in any of claims 1 to 8, wherein c is from 0.5 to 4.

10. A multimetal oxide as claimed in any of claims 1 to 9, wherein a + b + c is from 0.5 to 4.5.

11. A multimetal oxide as claimed in any of claims 1 to 10, wherein x is from 15 to 50.

12. A multimetal oxide as claimed in any of claims 1 to 11, wherein x is from 25 to 40.

13. A multimetal oxide as claimed in any of claims 1 to 12, wherein x is from 30 to 40.

14. A multimetal oxide as claimed in any of claims 1 to 7 and 10 to 13, wherein c is 0 and $M^1$ is exclusively W, and at least 25% of the vanadium contained are present as $V^{4+}$.

15. A process for the catalytic gas-phase oxidation of organic compounds, wherein the catalyst used is one whose active material is a multimetal oxide as claimed in any of claims 1 to 14.

16. A process as claimed in claim 15, which is the process for the catalytic gas-phase oxidation of acrolein to acrylic acid.

17. A catalyst whose active material consists of a multimetal oxide as claimed in any of claims 1 to 14.

18. Use of a multimetal oxide as claimed in any of claims 1 to 14 as the starting compound for the preparation of multimetal oxide materials containing Mo, V and at least one of the elements W, Nb, Ti, Zr, Hf, Ta, Cr, Si and Ge.

19. A process for the preparation of a multimetal oxide material containing Mo, V and at least one of the elements W, Nb, Ti, Zr, Hf, Ta, Cr, Si and Ge, wherein at least one multimetal oxide as claimed in any of claims 1 to 14 is used as the starting compound.

20. A finely divided mixture containing a finely divided multimetal oxide I as claimed in any of claims 1 to 14 and a finely divided multimetal oxide of the formula II

$$M^3_{12} \, Cu_d \, H_e \, O_y \qquad \text{(II)},$$

where

$M^3$ is Mo, W, V, Nb or Ta,

d is from 4 to 30,

e is from 0 to 20 and

y is a number which is determined by the valency and frequency of the elements in II other than oxygen.

21. A multimetal oxide material having at least two phases and of the formula III

$$[A]_p \, [B]_q \qquad \text{(III)};$$

wherein

$$A = (Mo_{12-a-b-c} \, V_a \, M^1_b \, M^2_c \, O_x) \, x \, \frac{12}{12\text{-}a\text{-}b\text{-}c} \qquad \text{(active phase)},$$

$$B = M^3_{12} \, Cu_d \, H_e \, O_y \qquad \text{(promoter phase)},$$

$M^1$ is W or Nb,

$M^2$ is Ti, Zr, Hf, Ta, Cr, Si or Ge,

a is from 0.1 to 6,

b is from 0 to 6,

c is from 0 to 6,

with the proviso that a + b + c is from 0.1 to 6,

x is a number which is determined by the valency and frequency of the elements in A other than oxygen,

$M^3$ is Mo, W, V, Nb or Ta,

d is from 4 to 30,

e is from 0 to 20,

y is a number which is determined by the valency and frequency of the elements in B other than oxygen,

p and q are numbers other than zero, whose ratio p/q is from 160:1 to 1:1,

which contains the moiety $[A]_p$ in the form of three-dimensional regions A which are delimited from their local environment owing to their chemical composition differing from their local environment and are of the chemical composition

$$A \qquad Mo_{12-a-b-c} V_a M^1_b M^2_c O_x$$

and the moiety $[B]_q$ in the form of three dimensional regions B which are delimited from their local environment owing to their chemical composition differing from their local environment and are of the chemical composition

$$B \qquad M^3_{12} Cu_d H_e O_y$$

where the regions A and B are distributed relative to one another as in a mixture of finely divided A and finely divided B and, with the use of CuKα radiation (λ = 1.54178 Å), the three-dimensional atomic arrangement of the regions A gives an X-ray powder diffraction spectrum (the intensity of the diffracted X-rays plotted as a function of twice the diffraction angle ($2\ominus$)) which, in the $2\ominus$ range from 5 to 50°, contains at least the following characteristic diffraction lines $A^1$, $A^3$, $A^5$, $A^9$ and $A^{10}$ but not more than the following diffraction lines $A^1$ to $A^{10}$:

| X-ray diffraction line | $2\ominus$ [°] |
|---|---|
| $A^1$ | 8.3 ± 0.7 |
| $A^2$ | 14.4 ± 0.7 |
| $A^3$ | 22.3 ± 0.2 |
| $A^4$ | 23.5 ± 0.7 |
| $A^5$ | 27.2 ± 0.4 |
| $A^6$ | 32.0 ± 0.8 |
| $A^7$ | 34.8 ± 0.6 |
| $A^8$ | 38.7 ± 0.5 |
| $A^9$ | 45.4 ± 0.4 |
| $A^{10}$ | 48.8 ± 0.4 |

**22.** A multimetal oxide material having at least two phases and of the formula III

$$[A]_p [B]_q \qquad\qquad (III),$$

wherein

$$A = (Mo_{12-a-b-c} V_a M^1_b M^2_c O_x) \cdot \frac{12}{12-a-b-c} \qquad \text{(active phase)},$$

$$B = M^3_{12} Cu_d H_e O_y \qquad \text{(promoter phase)},$$

$M^1$  is W or Nb,

$M^2$  is Ti, Zr, Hf, Ta, Cr, Si or Ge,

a    is from 0.1 to 6,

b    is from 0 to 6,

c    is from 0 to 6,

with the proviso that a + b + c is from 0.1 to 6,

x        is a number which is determined by the valency and frequency of the elements in A other than oxygen,

$M^3$  is Mo, W, V, Nb or Ta,

d  is from 4 to 30,

e  is from 0 to 20,

y  is a number which is determined by the valency and frequency of the elements in B other than oxygen,

p and q  are numbers other than zero, whose ratio p/q is from 160:1 to 1:1,

which contains the moiety $[A]_p$ in the form of three-dimensional regions A which are delimited from their local environment owing to their chemical composition differing from their local environment and are of the chemical composition

$$A \qquad Mo_{12-a-b-c} V_a M^1_b M^2_c O_x$$

and the moiety $[B]_q$ in the form of three dimensional regions B which are delimited from their local environment owing to their chemical composition differing from their local environment and are of the chemical composition

$$B \qquad M^3_{12} Cu_d H_e O_y$$

where the regions A and B are distributed relative to one another as in a mixture of finely divided A and finely divided B and a three-dimensional atomic arrangement of the regions A is that of the multimetal oxide as claimed in claim 4.

23. A multimetal oxide material as claimed in claim 21 or 22, whose regions B have the structure type of at least one of the copper molybdates listed in Table 1 below (the expression in brackets indicates the source of the associated X-ray diffraction fingerprint):

Table 1:

| | |
|---|---|
| $Cu_3(MoO_4)_2(OH)_2$ | (Lindgrenite, index card 36-405 of the JCPDS-ICDD index (1991)), |
| $Cu_4MoO_6O_{20}$ | (A. Moini et al., Inorg. Chem. 25(21) (1986) page 3782 to 3785), |
| $Cu_4Mo_5O_{17}$ | (Index card 39-181 of the JCPDS-ICDD index (1991)), |
| $Cu_6Mo_5O_{18}$ | (Index card 40-865 of the JCPDS-ICDD index (1991)), |
| $Cu_6Mo_4O_{15}$ | (Index card 35-17 of the JCPDS-ICDD index (1991)), |
| $CuMoO_4$ | (Index card 22-242 of the JCPDS-ICDD index (1991)), |
| $CuMoO_4$ | (Russian Journal of Inorganic Chemistry 36(7) (1991), 927-928, Table 1, $CuMoO_4$-III with distorted wolframite structure ($CuWO_4$, index card 21-307 of the JCPDS-ICDD index (1994)), |
| $CuMoO_4$ | (Index card 26-546 of the JCPDS-ICDD index (1991)), |
| HT-Cu molybdate | (DE-A 19 528 646), |
| $Cu_{4-x}Mo_3O_{12}$ | where x is from 0 to 0.25 (index cards 24-56 and 26-547 of the JCPDS-ICDD index (1991)), |
| $Cu_3Mo_2O_9$ | (Index cards 24-55 and 34-637 of the JCPDS-ICDD index (1991)) and |
| $Cu_2MoO_5$ | (Index cards 22-607 of the JCPDS-ICDD index (1991)). |

24. A multimetal oxide material as claimed in any of claims 21 to 23, whose regions A and B have maximum diameters of from > 0 to 300 μm.

25. A multimetal oxide material as claimed in any of claims 21 to 23, whose regions A and B have maximum diameters of from 1 to 30 μm.

**26.** A process for the preparation of a catalyst containing multimetal oxide materials as claimed in any of claims 21 to 23 as active material, wherein at least one finely divided multimetal oxide I as claimed in any of claims 1 to 14 and at least one finely divided multimetal oxide of the formula II

$$M^3_{12} \, Cu_d \, H_e \, O_y \qquad (II),$$

where

$M^3$ is Mo, W, V, Nb or Ta,

d is from 4 to 30,

e is from 0 to 20 and

y is a number which is determined by the valency and frequency of the elements in II other than oxygen,

are each separately preformed, the separately preformed finely divided multimetal oxides I and II are mixed to give a thorough dry mixture and the latter is molded.

**27.** A process for the catalytic gas-phase oxidation of organic compounds, wherein the catalyst used is one whose active material is a multimetal oxide material as claimed in any of claims 21 to 25.

**28.** A process as claimed in claim 27, which is a process for the catalytic gas-phase oxidation of acrolein to acrylic acid.

**29.** A multiphase multimetal oxide material which contains phases comprising multimetal oxides I as claimed in any of claims 1 to 14 in finely divided homogeneous distribution having maximum diameters of from > 0 to 300 µm.

**Revendications**

**1.** Oxydes multimétalliques de formule générale I

$$Mo_{12-a-b-c} \, V_a \, M^1_b \, M^2_c \, O_x \qquad (I),$$

dans laquelle les variables ont les significations suivantes :

$M^1 =$    W et/ou Nb,
$M^2 =$    Ti, Zr, Hf, Ta, Cr, Si et/ou Ge,
a =    0,1-6,
b =    0-6,
c =    0-6,

étant spécifié que a+b+c = 0,1-6 et

x =    un nombre déterminé par la fréquence et la valence dans I des éléments autres que l'oxygène,

caractérisés en ce que leur arrangement atomique dans l'espace fournit un spectre de diffraction des rayons X (l'intensité A du rayon X diffracté en fonction du double de l'angle de diffraction (2Θ)), par utilisation de rayons Cu-Kα (λ = 1,54178 Å) sur leur poudre, qui contient, dans le domaine 2Θ de 5° à 50°, au moins les raies de diffraction caractéristiques suivantes $A^1$, $A^3$, $A^5$, $A^9$ et $A^{10}$ et au plus les raies de diffraction caractéristiques suivantes $A^1$ à $A^{10}$ :

| raie de diffraction des rayons X | 2 Θ [°] |
|---|---|
| $A^1$ | 8,3 ± 0,7 |

(suite)

| raie de diffraction des rayons X | $2\ominus[°]$ |
|---|---|
| $A^2$ | $14{,}4 \pm 0{,}7$ |
| $A^3$ | $22{,}3 \pm 0{,}2$ |
| $A^4$ | $23{,}5 \pm 0{,}7$ |
| $A^5$ | $27{,}2 \pm 0{,}4$ |
| $A^6$ | $32{,}0 \pm 0{,}8$ |
| $A^7$ | $34{,}8 \pm 0{,}6$ |
| $A^8$ | $38{,}7 \pm 0{,}5$ |
| $A^9$ | $45{,}4 \pm 0{,}4$ |
| $A^{10}$ | $48{,}8 \pm 0{,}4$ |

2.  Oxydes multimétalliques selon la revendication 1, où les raies de diffraction $A^1$ à $A^{10}$ présentent les amplitudes relatives suivantes ($I_A^{rel}[\%]$):

|  | $I_A^{rel}[\%]$ |
|---|---|
| $A^1$ | $7 \pm 5$ |
| $A^2$ | $5 \pm 5$ |
| $A^3$ | $100$ |
| $A^4$ | $40 \pm 40$ |
| $A^5$ | $70 \pm 40$ |
| $A^6$ | $25 \pm 25$ |
| $A^7$ | $20 \pm 20$ |
| $_A 8$ | $10 \pm 10$ |
| $A^9$ | $25 \pm 15$ |
| $A^{10}$ | $35 \pm 20$ |

3.  Oxydes multimétalliques selon la revendication 1 ou 2, où le spectre de diffraction des rayons X au Cu-K$\alpha$ sur la poudre ne présente pas de raies de diffraction hautement décomposées, dont la largeur à mi hauteur (dans le domaine 2$\Theta$) est inférieure à $0{,}25°$.

4.  Oxydes multimétalliques selon l'une quelconque des revendications 1 à 3, de formule générale I

$$Mo_{12-a-b-c} \, V_a \, M^1_b \, M^2_c \, O_x \qquad (I),$$

dans laquelle les variables ont les significations suivantes :

$M^1 =$ W et/ou Nb,
$M^2 =$ Ti, Zr, Hf, Ta, Cr, Si et/ou Ge,
$a =$ 0,1-6,
$b =$ 0-6,
$c =$ 0-6,

étant spécifié que a+b+c = 0,1-6 et

$x =$ un nombre déterminé par la fréquence et la valence dans I des éléments autres que l'oxygène,

caractérisés en ce que leur arrangement atomique dans l'espace correspond à celui de l'oxyde multimétallique $Mo_{8,54}V_{2,47}W_{0,99}O_{33,35}$ pouvant être obtenu comme suit :

33,746 kg d'heptamolybdate d'ammonium hydraté (teneur en $MoO_3$ : 81,8% en poids, composition idéale : $(NH_4)_6Mo_7O_{24} \times 4 H_2O$), 6,576 kg de métavanadate d'ammonium (teneur en $V_2O_5$ : 76,5% en poids, composition idéale : $NH_4VO_3$), 5,764 kg de paratungstate d'ammonium hydraté (teneur en $WO_3$ : 89,0% en poids, composition idéale : $(NH_4)_{10}W_{12}O_{41} \times 7 H_2O$) et 7,033 kg d'acétate d'ammonium (teneur en $CH_3COONH_4$ : 97,0% en poids, composition idéale : $CH_3COONH_4$) sont dissous sous agitation les uns après les autres, dans l'ordre mentionné, à une température de 90°C dans 250 l d'eau. La solution résultante de couleur jaune à orange est refroidie à 80°C puis séchée par pulvérisation avec une température d'entrée de 300°C et une température de sortie de 110°C ;

800 g de la poudre de pulvérisation obtenue sont malaxés pendant 1 heure dans un malaxeur ayant un volume utile de 2,5 l (Type LUK 2,5 de la société Werner et Pfleiderer, 7000 Stuttgart, DE) avec ajout de 250 g d'eau. Parmi ces 250 g d'eau, 180 g sont ajoutés pendant les 10 premières minutes du malaxage et 70 g sont ajoutés pendant les 50 minutes restantes. Le produit malaxé résultant, humide et sous forme de mottes, est séché pendant 15 h à une température de 110°C puis comprimé à travers un tamis ayant une ouverture de maille de 5 mm ; 100 g du granulat résultant sont ensuite calcinés dans un four sphérique horizontal tournant ayant un volume sphérique en quartz chauffé de manière isotherme de 1 l et une vitesse de rotation de 12 tours par minute, où les conditions de calcination sont les suivantes :

1ère étape : chauffer de façon continue le granulat utilisé de 25 à 275°C en 50 minutes ;
2ème étape : chauffer de façon continue le granulat utilisé de 275 à 325°C en 30 minutes ;
3ème étape : maintenir le granulat utilisé à 325°C pendant 4 h ;
4ème étape : chauffer de façon continue le granulat utilisé de 325 à 400°C en 30 minutes ;
5ème étape : maintenir le granulat utilisé à 400°C pendant 10 minutes ; puis le chauffage extérieur du four tournant sphérique est interrompu et ce dernier est refroidi par soufflage extérieur avec de l'air ambiant, ce qui permet de refroidir le granulat jusqu'à 25°C en 5 heures ;

pendant chacune des étapes de calcination, l'espace intérieur du four tournant sphérique est traversé parallèlement à l'axe de rotation par des mélanges gazeux présentant les compositions suivantes (conditions normales (N) = 1 atm, 25°C) :

| | |
|---|---|
| 1ère, 2ème et 3ème étapes: | 3,6 Nl/h d'air, 1,5 Nl/h de NH3 et 44,9 Nl/h de N2 (débit gazeux total : 50 Nl/h) ; |
| 4ème et 5ème étapes et phase de refroidissement: | 3,6 Nl/h d'air et 44,9 Nl/h de N2 (débit gazeux total : 48,5 Nl/h). |

5. Oxydes multimétalliques selon l'une quelconque des revendications 1 à 4, où a = 0,5-4,5.

6. Oxydes multimétalliques selon l'une quelconque des revendications 1 à 5, où b = 0,1-6.

7. Oxydes multimétalliques selon l'une quelconque des revendications 1 à 6, où b = 0,5-4.

8. Oxydes multimétalliques selon l'une quelconque des revendications 1 à 7, où c = 0,1-6.

9. Oxydes multimétalliques selon l'une quelconque des revendications 1 à 8, où c = 0,5-4.

10. Oxydes multimétalliques selon l'une quelconque des revendications 1 à 9, où a+b+c = 0,5-4,5.

11. Oxydes multimétalliques selon l'une quelconque des revendications 1 à 10, où x = 15-50.

12. Oxydes multimétalliques selon l'une quelconque des revendications 1 à 11, où x = 25-40.

13. Oxydes multimétalliques selon l'une quelconque des revendications 1 à 12, où x = 30-40.

14. Oxydes multimétalliques selon l'une quelconque des revendications 1 à 7 et 10 à 13, où c = 0 et $M^1$ est exclusivement W, et où au moins 25% du vanadium contenu est sous forme de $V^{4+}$.

15. Procédé d'oxydation catalytique en phase gazeuse de composés organiques, caractérisé en ce que l'on utilise un catalyseur dont la masse active est un oxyde multimétallique selon l'une quelconque des revendications 1 à 14.

**16.** Procédé selon la revendication 15, caractérisé en ce qu'il s'agit du procédé d'oxydation catalytique en phase gazeuse de l'acroléine en acide acrylique.

**17.** Catalyseur dont la masse active est constituée d'un oxyde multimétallique selon l'une quelconque des revendications 1 à 14.

**18.** Utilisation d'oxydes multimétalliques selon l'une quelconque des revendications 1 à 14 en tant que matériaux de départ pour la préparation de masses d'oxydes multimétalliques contenant Mo, V et au moins un des éléments du groupe formé par W, Nb, Ti, Zr, Hf, Ta, Cr, Si et/ou Ge.

**19.** Procédé de préparation de masses d'oxydes multimétalliques contenant Mo, V et au moins un des éléments du groupe formé par W, Nb, Ti, Zr, Hf, Ta, Cr, Si et/ou Ge, caractérisé en ce que l'on utilise au moins un oxyde multimétallique selon l'une quelconque des revendications 1 à 14 en tant que matériau de départ.

**20.** Mélange finement divisé, contenant un oxyde multimétallique I finement divisé selon l'une quelconque des revendications 1 à 14 ainsi qu'un oxyde multimétallique finement divisé de formule générale II

$$M^3_{12} \, Cu_d \, H_e \, O_y \qquad \text{(II)},$$

avec

M$^3$ = Mo, W, V, Nb et/ou Ta,
d = 4-30,
e = 0-20, et
y = un nombre déterminé par la fréquence et la valence dans II des éléments autres que l'oxygène.

**21.** Masses d'oxydes multimétalliques au moins biphasiques de formule générale III

$$[A]_p \, [B]_q \qquad \text{(III)},$$

dans laquelle les variables ont les significations suivantes :

$$A = (Mo_{12-a-b-c} \, V_a \, M^1_b \, M^2_c \, O_x) \times \frac{12}{12-a-b-c} \qquad \text{(phase active)},$$

$$B = M^3_{12} \, Cu_d \, H_e \, O_y \qquad \text{(phase de promoteur)},$$

M$^1$ = W et/ou Nb,
M$^2$ = Ti, Zr, Hf, Ta, Cr, Si et/ou Ge,
a = 0,1-6,
b = 0-6,
c = 0-6,

étant spécifié que a+b+c = 0,1-6 et

x = un nombre déterminé par la fréquence et la valence dans A des éléments autres que l'oxygène,
M$^3$ = Mo, W, V, Nb et/ou Ta,
d = 4-30,
e = 0-20, et
y = un nombre déterminé par la fréquence et la valence dans B des éléments autres que l'oxygène,
p, q = des nombres différents de zéro, dont le rapport p/q vaut de 160:1 à 1:1,

qui contiennent la partie [A]$_p$ sous forme de domaines A, ayant une extension tridimensionnelle, séparés de leur environnement local du fait de leur composition chimique, qui est différente de celle de leur environnement local, et ayant la composition chimique

$$A \qquad Mo_{12-a-b-c} \, V_a \, M^1_b \, M^2_c \, O_x$$

et la partie $[B]_q$ sous forme de domaines B, ayant une extension tridimensionnelle, séparés de leur environnement local du fait de leur composition chimique, qui est différente de celle de leur environnement local, et ayant la composition chimique :

$$B \qquad M^3_{12} \, Cu_d \, H_e \, O_y$$

où les domaines A, B sont répartis l'un par rapport à l'autre comme dans un mélange constitué de A finement divisé et de B finement divisé, et l'arrangement atomique dans l'espace des domaines A fournit un spectre de diffraction des rayons X sous forme de poudre (l'intensité A du rayon X diffracté en fonction du double de l'angle de diffraction (2Θ)), par utilisation de rayons Cu-K$\alpha$ ($\lambda$ = 1,54178 Å) sur leur poudre, qui contient, dans le domaine 20 de 5° à 50°, au moins les raies de diffraction caractéristiques suivantes $A^1$, $A^3$, $A^5$, $A^9$ et $A^{10}$ et au plus les raies de diffraction caractéristiques suivantes $A^1$ à $A^{10}$ :

| raie de diffraction des rayons X | 2 Θ [°] |
|---|---|
| $A^1$ | 8,3 ± 0,7 |
| $A^2$ | 14,4 ± 0,7 |
| $A^3$ | 22,3 ± 0,2 |
| $A^4$ | 23,5 ± 0,7 |
| $A^5$ | 27,2 ± 0,4 |
| $A^6$ | 32,0 ± 0,8 |
| $A^7$ | 34,8 ± 0,6 |
| $A^8$ | 38,7 ± 0,5 |
| $A^9$ | 45,4 ± 0,4 |
| $A^{10}$ | 48,8 ± 0,4 |

**22.** Masses d'oxydes multimétalliques au moins biphasiques de formule générale III

$$[A]_p \, [B]_q \qquad\qquad\qquad (III),$$

dans laquelle les variables ont les significations suivantes :

$$A = (Mo_{12-a-b-c} \, V_a \, M^1_b \, M^2_c \, o_x) \times \frac{12}{12-a-b-c} \qquad \text{(phase active)},$$

$$B = M^3_{12} \, Cu_d \, H_e \, O_y \qquad \text{(phase de promoteur)},$$

$M^1$ = W et/ou Nb,
$M^2$ = Ti, Zr, Hf, Ta, Cr, Si et/ou Ge,
a = 0,1-6,
b = 0-6,
c = 0-6,

étant spécifié que a+b+c = 0,1-6 et

x = un nombre déterminé par la fréquence et la valence dans A des éléments autres que l'oxygène,

$M^3 =$ Mo, W, V, Nb et/ou Ta,

$d =$ 4-30,

$e =$ 0-20, et

$y =$ un nombre déterminé par la fréquence et la valence dans B des éléments autres que l'oxygène,

$p, q =$ des nombres différents de zéro, dont le rapport p/q vaut de 160:1 à 1:1,

qui contiennent la partie $[A]_p$ sous forme de domaines A, ayant une extension tridimensionnelle, séparés de leur environnement local du fait de leur composition chimique, qui est différente de celle de leur environnement local, et ayant la composition chimique

$$A \qquad Mo_{12-a-b-c} \, V_a \, M^1_b \, M^2_c \, O_x$$

et la partie $[B]_q$ sous forme de domaines B, ayant une extension tridimensionnelle, séparés de leur environnement local du fait de leur composition chimique, qui est différente de celle de leur environnement local, et ayant la composition chimique :

$$B \qquad M^3_{12} \, Cu_d \, H_e \, O_y$$

où les domaines A, B sont répartis l'un par rapport à l'autre comme dans un mélange constitué de A finement divisé et de B finement divisé, et l'arrangement atomique dans l'espace des domaines A est celui de l'oxyde multimétallique selon la revendication 4.

**23.** Masses d'oxydes multimétalliques selon la revendication 21 ou 22, dont les domaines B présentent le type de structure d'au moins un des molybdates de cuivre rassemblés dans le tableau 1 suivant (l'expression entre parenthèses redonne l'origine des spectres de diffraction des rayons X correspondants) :

Tableau 1:

| | |
|---|---|
| $Cu_3(MoO_4)_2(OH)_2$ | (Lindgrenit, fiche 36-405 du fichier JCPDS-ICDD (1991)), |
| $Cu_4MoO_6O_{20}$ | (A. Moini et al., Inorg. Chem. 25(21) (1986) p. 3782-3785) |
| $Cu_4Mo_5O_{17}$ | (fiche 39-181 du fichier JCPDS-ICDD (1991)), |
| $Cu_6Mo_5O_{18}$ | (fiche 40-865 du fichier JCPDS-ICDD (1991)), |
| $Cu_6Mo_4O_{15}$ | (fiche 35-17 du fichier JCPDS-ICDD (1991)), |
| $CuMoO_4$ | (fiche 22-242 du fichier JCPDS-ICDD (1991)), |
| $CuMoO_4$ | (Russian Journal of Inorganic Chemistry, 36(7), 1991, p.927-928, tableau 1, $CuMoO_4$-III avec structure de tungstite déformée ($CuWO_4$, fiche 21-307 du fichier JCPDS-ICDD (1994)), |
| $CuMoO_4$ | (fiche 26-546 du fichier JCPDS-ICDD (1991)), |
| molybdate de Cu-HT | (DE -A 19528646) |
| $Cu_{4-x}Mo_3O_{12}$ | avec x=0-0,25 (fiches 24-56 et 26-547 du fichier JCPDS-ICDD (1991)), |
| $Cu_3Mo_2O_9$ | (fiches 24-55 et 34-637 du fichier JCPDS-ICDD (1991)), |
| $Cu_2MoO_5$ | (fiche 22-607 du fichier JCPDS-ICDD (1991)), |

**24.** Masses d'oxydes multimétalliques selon l'une quelconque des revendications 21 à 23, dont les domaines A et B présentent un diamètre maximum dans la gamme > 0 à 300 μm.

**25.** Masses d'oxydes multimétalliques selon l'une quelconque des revendications 21 à 23, dont les domaines A et B présentent un diamètre maximum dans la gamme 1 à 30 μm.

**26.** Procédé de préparation de catalyseurs à masses d'oxydes multimétalliques selon l'une quelconque des revendications 21 à 23 en tant que masse active, caractérisé en ce que l'on prépare de façon séparée au moins un oxyde multimétallique I finement divisé selon l'une quelconque des revendications 1 à 14 et au moins un oxyde multimétallique finement divisé de formule générale II

$$M^3_{12} \, Cu_d \, H_e \, O_y \qquad\qquad (II),$$

avec

M$^3$ = Mo, W, V, Nb et/ou Ta,
d = 4-30,
e = 0-20, et
y = un nombre déterminé par la fréquence et la valence dans II des éléments autres que l'oxygène,

puis on mélange les oxydes multimétalliques finement divisés I et II préparés de façon séparée en un mélange intime et sec, et l'on façonne le mélange intime et sec.

27. Procédé d'oxydation catalytique en phase gazeuse de composés organiques, caractérisé en ce que l'on utilise un catalyseur dont la masse active est une masse d'oxyde multimétallique selon l'une quelconque des revendications 21 à 25.

28. Procédé selon la revendication 27, caractérisé en ce qu'il s'agit de l'oxydation catalytique en phase gazeuse de l'acroléine en acide acrylique.

29. Masses d'oxydes multimétalliques à plusieurs phases présentant des phases à base d'oxydes multimétalliques I selon l'une quelconque des revendications 1 à 14 distribués de façon finement divisés et homogènes avec un diamètre maximum dans la gamme > 0 à 300 µm.

# FIG.1

# FIG.2

# FIG.3

# FIG.4

# FIG.5

# FIG.6

# FIG.7

# FIG.8

# FIG.9

# FIG.10

# FIG.11

EP 0 774 297 B1

# FIG.12

# FIG.13

# FIG.14

# FIG.15

# FIG.16

# FIG.17

# FIG.18

# FIG.19